# EUROPEAN PATENT SPECIFICATION

(11) **EP 3 862 429 B1**
(45) Date of publication and mention of the grant of the patent: **21.08.2024**
(21) Application number: 19869268.3
(22) Date of filing: 19.09.2019
(51) Int. Cl.: C12N 13/00, C12N 5/077

(54) **COMPOSITION FOR EXTENDING TELOMERE OF CELL AND PREPARATION METHOD THEREFOR**
ZUSAMMENSETZUNG ZUR VERLÄNGERUNG DES TELOMERS EINER ZELLE UND HERSTELLUNGSVERFAHREN DAFÜR
COMPOSITION POUR ÉTENDRE LE TÉLOMÈRE DE CELLULE ET PROCÉDÉ DE PRÉPARATION ASSOCIÉ

(30) Priority: 02.10.2018 KR 20180117265
(43) Date of publication of application: 11.08.2021
(73) Proprietor: STEMON Inc., Gyeonggi-do 13486 (KR)
(72) Inventor: LEE, Yong Seung, Suwon-si Gyeonggi-do 16591 (KR)
(74) Representative: M. Zardi & Co S.A.
(86) International application number: PCT/KR2019/012150
(87) International publication number: WO 2020/071665

(56) References cited:
- WO-A1-2017/091702
- WO-A1-2018/057542
- WO-A1-2018/057542
- KR-A- 20170 106 149
- KR-B1- 101 855 967
- US-A1- 2014 242 154
- US-A1- 2014 242 155
- ANNA GUTKIN ET AL: "Tumor cells derived exosomes contain hTERT mRNA and transform nonmalignant fibroblasts into telomerase positive cells", ONCOTARGET, vol. 7, no. 37, 13 September 2016 (2016-09-13), United States, pages 59173 - 59188, XP055710168, ISSN: 1949-2553, DOI: 10.18632/oncotarget.10384
- GUTKIN, A. ET AL.: "Tumor cells derived exosomes contain hTERT mRNA and transform nonmalignant fibroblasts into telomerase positive cells", ONCOTARGET, vol. 7, no. 37, 2 July 2016 (2016-07-02), pages 59173 - 59188, XP55710168
- MURILLO-ORTIZ, B. ET AL.: "Increased telomere length and proliferative potential in peripheral blood mononuclear cells of adults of different ages stimulated with concanavalin A", BMC GERIATRICS, vol. 13, 2013, pages 1 - 5, XP021163371

## Description

### [Technical Field]

The present invention relates to exosomes for elongating telomeres of cells, and encompasses in particular a production method therefor, and more particularly, relates to exosomes for elongating telomeres of cells, which contain an RNA or protein of at least one gene selected from among TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin, and encompasses in particular a method of inducing telomere-elongating exosomes from cells by providing physical stimulation directly or indirectly to the cells.

### [Background Art]

Telomeres are DNA repeat structures (TTAGGG in humans) at both ends of chromosomes. Telomeres bind to the shelterin complex to form a protective cap, which regulates the multiplication ability of cells and prevents binding between chromosomes and loss of genetic information during cell division. Since DNA polymerase cannot completely amplify the 3' end, telomeres are shortened by 30 to 200 bp for each cell division. When telomeres become shorter than the threshold value and become closer to the coding DNA and the loop structure of the telomeres cannot be maintained, the exposed telomeres are recognized by the p53 or p16INK4a signaling pathways, so that cell division stops and the cells die due to senescence.

Telomeres may be elongated by the reverse transcriptase telomerase, and the human telomerase complex is composed of TERC, which is an RNA molecule that acts as a template for telomere synthesis, TERT, which is a catalytic subunit, and telomerase-associated proteins such as DKC1 and TEP1. In normal cases, somatic cells have little telomerase activity, and high telomerase activity is detected only in stem cells and progenitor cells that require active division ability.

When the division of multiple cells is stopped as the telomeres are shortened, various problems may arise, and in particular, various symptoms represented by aging and degeneration may arise. It was already known that diseases that cause problems in the regeneration of bone marrow cells, such as congenital dyskeratosis congenita in which skin tissue is degenerated, and aplastic anemia in which blood cells counts are low, arise due to abnormalities in telomeres maintenance, such as mutations in telomerase or Shelterin complex-related genes containing the same. In recent years, studies have been reported that telomeres in patients suffering from aging-related diseases such as hypertension, metabolic syndrome, diabetes, and dementia are shorter than those in normal people, and thus studies on the possibility of slowing aging by increasing the length of telomeres have attracted attention. In addition, it has been shown that regenerative activities can be actively performed by increasing telomerase activity in somatic cells, which are generally known to have little telomerase activity. In particular, it has been reported that the increase in telomerase activity is effective when it is necessary to supplement various tissues such as the myocardium, liver, cornea, skin, blood vessels, cartilage and bone, immune cells, blood cells, etc. due to bums, injuries, aging and diseases. However, studies have also been reported that a number of cancer cells occurred in mice in which a gene capable of extending the length of telomeres was continuously overexpressed, suggesting that long telomeres become a risk factor for cancer. Therefore, it can be expected that, if telomeres can be temporarily elongated, this telomere elongation can alleviate various symptoms associated with aging and degeneration without the risk of cancer development, and can be particularly useful in the field of regenerative medicine.

According to the various utilities expected as described above, various methods for elongating telomeres have been developed. US Patent Application Publication No. 2014-242154 discloses exosomes including an RNA encoding TERT and compositions thereof, for extending cell telomeres. Oncotarget, 2016, vol.7(37), pp. 59173-59188 describes tumor-cell derived exosomes containing hTERT mRNA, used to transform non-malignant fibroblasts into telomerase-positive cells. International application WO2018057542 relates to the extension of telomere length by use of a composition comprising e.g. cardiosphere-derived extracellular vesicles which contained active telomerase. International application WO2017091702 discloses a method to promote telomere extension by contacting cells with a composition comprising a RNA encoding a telomerase, preferably carried by an exosome. All these documents are silent on specific methods to produce exosomes. Korean application 20170106149 relates to the reprogramming of cells e.g. using exosomes produced by ultrasonicationg cells and extraction of the exosomes from the medium; the document is however silent on telomeres and related genes. US Patent Application Publication No. 2018-0280413 discloses a pharmaceutical composition containing a compound for enhancing telomerase activity. However, in this case, a process of synthesizing the compound is required, and the composition obtainable through chemical synthesis requires that the starting material required for synthesis is purified. In addition, various by-products harmful to the environment are produced during the synthesis process, and the compound has a risk of producing by-products in *in vivo* metabolic processes. European Patent Publication No. 2959005 discloses an RNA encoding telomerase. In this case, synthesis and purification processes are also required, and since the RNA is hydrophilic, a delivery vehicle is additionally required to deliver the RNA into cells. Therefore, there is a need for a composition for telomere elongation that can be produced more simply, is less harmful, and has good efficiency.

### [Disclosure]

### [Technical Problem]

The present invention has been made in order to solve the above-described problems, and exosomes produced according to the method of the present invention contain an RNA or protein of at least one gene selected from among TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin.

The method for producing exosomes for elongating telomeres of cells object of the present invention comprises steps described by claim 1.

Technical problems to be achieved by the present invention are not limited to the above-mentioned technical problem, and other technical problems which are not mentioned herein will be clearly understood by those skilled in the art from the following description.

### [Technical Solution]

As a technical means for achieving the above-described technical problem, exosomes for elongating telomeres of cells produced according to the method of the present invention contain an RNA or protein of at least one gene selected from among TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin.

Here, the exosomes are induced by providing ultrasound stimulation directly to the cells, wherein providing said stimulation directly to the cells comprises applying the stimulation to the medium containing the cells, in particular by mixing the cells and the medium and then providing physical stimulation to the mixture; or providing physical stimulation to the cells and then mixing the cells and the medium; or providing physical stimulation to the cells and then mixing the cells and the medium, followed by providing physical stimulation to the mixture; or providing physical stimulation to the medium and then mixing the medium and the cells, followed by providing physical stimulation to the mixture; or providing physical stimulation to each of the cells and the medium and then mixing the cells and the medium; or providing physical stimulation to each of the cells and the medium and then mixing the cells and the medium, followed by providing physical stimulation to the mixture.

Providing the ultrasound stimulation directly to the cells is performed at an ultrasound intensity of 0.1 to 3 W/cm² and a frequency of 20 kHz to 20 MHz for a duration of 0.1 seconds to 20 minutes.

The exosomes may contain 150 ng or more of total RNA in 1 × 10⁸ exosome particles.

The exosomes may contain 1 µg or more of total RNA in 1 × 10⁸ exosome particles.

When cells are treated with the exosomes, telomerase activity in the cells may increase.

When cells are treated with the exosomes, β-galactosidase activity in the cells may decrease.

The above-described exosomes can be included in a composition having a concentration of 10⁶ to 10¹⁴ exosomes/ml.

Overall, the method object of the present invention comprises the steps of: a) providing direct ultrasound stimulation to cells at 0.1 to 3 W/cm2 and a frequency of 20 kHz to 20 MHz for a duration of 0.1 seconds to 20 minutes; b) culturing a mixture of the cells provided in step a) and a medium for a predetermined time; and c) isolating exosomes from the mixture, where in step a) the providing direct ultrasound stimulation to cells is applying the ultrasound stimulation to a medium containing the cells; wherein an expression level of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the isolated exosomes is higher than that in the exosomes secreted from the cells before being subjected to the method.

The step of providing the ultrasound stimulation directly to the cells may be performed by any one method selected from among: mixing the cells and the medium and then providing physical stimulation to the mixture; or providing physical stimulation to the cells and then mixing the cells and the medium; or providing physical stimulation to the cells and then mixing the cells and the medium, followed by providing physical stimulation to the mixture; or providing physical stimulation to the medium and then mixing the medium and the cells, followed by providing physical stimulation to the mixture; or providing physical stimulation to each of the cells and the medium and then mixing the cells and the medium; or providing physical stimulation to each of the cells and the medium and then mixing the cells and the medium, followed by providing physical stimulation to the mixture.

Providing the ultrasound stimulation directly to the cells may be performed at an ultrasound intensity of 0.1 to 3 W/cm² and a frequency of 20 kHz to 20 MHz for a duration of 0.1 seconds to 20 minutes.

The cells may be selected from the group consisting of mammalian stem cells, progenitor cells, fibroblasts, keratinocytes or organ tissue cells.

The medium may be selected from a culture medium or a differentiation-inducing medium.

The culturing of the mixture may be performed for 1 hour to 10 days.

The step of isolating the exosomes may be performed using one or more of ultracentrifugation, density gradient separation, filtration, size exclusion chromatography, immunoaffinity separation, precipitation, and microfluidic separation.

The step of isolating the exosomes may comprise steps of: centrifuging the mixture after the culturing to obtain a supernatant; filtering the supernatant through a filter to obtain a filtrate; and concentrating the filtrate.

The expression level of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the isolated exosomes may be higher than that in exosomes secreted from the cells before being subjected to the production method.

When cells are treated with the isolated exosomes, telomerase activity in the cells may increase.

When cells are treated with the isolated exosomes, β-galactosidase activity in the cells may decrease.

When cells are treated with the isolated exosomes obtained in the method for producing exosomes for elongating telomeres of cells, migration of the cells may increase.

When tissue is treated with the isolated exosomes obtained in the method for producing exosomes for elongating telomeres of cells, regeneration of the tissue may be promoted.

When a wound is treated with the isolated exosomes obtained in the method for producing exosomes for elongating telomeres of cells, healing of the wound may be promoted.

When a scar is treated with the isolated exosomes obtained in the method for producing exosomes for elongating telomeres of cells, healing of the scar may be promoted.

When cells are treated with the isolated exosomes obtained in the method for producing exosomes for elongating telomeres of cells, the exosomes may have an anti-senescence effect on the cells.

### [Advantageous Effects]

The composition for elongating telomeres of cells is simpler to produce than the above-described conventional composition for elongating telomeres. That is, the compound in the conventional composition is chemically synthesized, and this chemical synthesis has no specificity and requires a long and complex process. In contrast, the invention according to claim 1 is based on a simple process of treating the cells with physical stimulation, and includes isolating the secreted exosomes from the conditioned medium of the cells, and thus the process thereof is simple. In addition, since cells are surrounded by a double lipid membrane, the intracellular endocytosis of hydrophilic nucleic acid, which is required for the nucleic acid exhibits an effect, is difficult without a delivery vehicle capable of delivering the nucleic acid into cells, and thus a step of loading the nucleic acid into the delivery vehicle is additionally required. However, according to the method for producing exosomes according to the present invention, telomere elongation-related factors are obtained in a form surrounded by a lipid membrane, there is no need to perform additional loading.

In addition, the composition for elongating telomeres of cells is superior in terms of efficiency and application method compared to the previously disclosed composition. That is, the above-described compound for increasing telomerase activity needs to be injected into the body at a high concentration so that it exhibits a sufficient effect, whereas the exosomes for elongating telomeres of cells obtained by the method of the present invention may exhibit an effect by applying a small amount of the exosomes by a non-invasive method such as topical application.

In addition, since the method for producing exosomes for elongating telomeres of cells according to one embodiment of the present invention induces secretion of a large amount of exosomes containing large amounts of telomere elongation-related factors, it is advantageous in terms of the high-yield production of exosomes containing useful substances from cells.

In addition, the exosomes for elongating telomeres obtained by the method of the present invention have high safety. That is, the conventional compound may pose additional risks such as toxicity in the metabolic process, and particularly, in the above-described conventional technology, the dosage of the compound is quite high (the order of mg per kg weight of a subject animal, and waste that burden the environment is also generated in the synthesis process. However, this burden is relatively small in the method according to the present invention.

In addition, it was confirmed that the exosomes for elongating telomeres of cells obtained by the method of the present invention induce cell division and have anti-aging and tissue regeneration effects, in addition to elongating telomeres. Thereby, it is expected that the exosomes produced according to the present invention can ameliorate and prevent not only problems caused by shortening of telomeres, but also various diseases and conditions associated with aging and tissue regeneration.

It is to be understood that the effects of the present invention are not limited to the above-described effects, and include all effects that may be deduced from the features described in the detailed description of the invention or the claims.

### [Description of Drawings]

FIG. 1 shows data obtained by analyzing telomere length changes depending on the type of cells after direct ultrasound stimulation treatment and culture according to Example 1 of the present invention.
FIG. 2 shows data obtained by analyzing telomere length changes caused by each physical stimulation treatment after ultrasound stimulation treatment and culture according to Example 1 of the present invention, heat stimulation treatment and culture according to Example 2 (reference), and light-emitting diode laser light stimulation treatment and culture according to Example 3 (reference).
FIG. 3 shows data obtained by analyzing telomere length changes after indirect ultrasound stimulation treatment and culture according to Example 5 of the present invention.
FIG. 4 shows data obtained by analyzing telomere length changes depending on the kind of medium after ultrasound treatment and culture according to Example 1 of the present invention.
FIG. 5 shows data obtained by analyzing telomere length changes depending on the nu5/10dlfmber of ultrasound treatments after ultrasound treatment and culture according to Example 1 of the present invention.
FIG. 6 shows data obtained by analyzing changes in TERT gene expression depending on the type of cells after ultrasound treatment and culture of the cells according to Example 1 of the present invention.
FIG. 7 shows data obtained by analyzing changes in TERT gene expression in CB-HDFs and Adipo-MSCs after ultrasound treatment and culture of the CB-HDFs and Adipo-MSC cells according to Example 1 of the present invention.
FIG. 8 shows data obtained by analyzing changes in β-catenin gene expression in CB-HDFs and Adipo-MSC cells after ultrasound treatment and culture of the CB-HDFs and Adipo-MSC cells according to Example 1 of the present invention.
FIG. 9 shows data obtained by analyzing telomerase activity changes in CB-HDFs and Adipo-MSC cells after ultrasound treatment and culture of the CB-HDFs and Adipo-MSC cells according to Example 1 of the present invention.
FIG. 10 shows data obtained by performing telomere FISH for CB-HDFs and Adipo-MSCs after ultrasound treatment and culture according to Example 1 of the present invention.
FIG. 11 shows data obtained by performing fluorescence staining for TERT and Ki67 in CB-HDFs after ultrasound treatment and culture of the CB-HDFs according to Example 1 of the present invention.
FIG. 12 shows data obtained by analyzing senescence-related β-galactosidase activity in CB-HDFs after ultrasound treatment and culture of the CB-HDFs according to Example 1 of the present invention.
FIG. 13 shows data obtained by analyzing the expression of an exosome marker in CB-HDFs treated with ultrasound according to Example 5 of the present invention.
FIG. 14 shows data obtained by performing Nanosight analysis depending on the particle size of the exosomes secreted from CB-HDFs according to Example 5 of the present invention.
FIG. 15 shows data obtained by performing Nanosight analysis for the total number of the exosomes secreted from CB-HDFs according to Example 5 of the present invention.
FIG. 16 shows data obtained by analyzing the amounts of total RNA and total protein in the exosomes produced according to Example 5 of the present invention.
FIG. 17 shows data obtained by performing gene ontology analysis for RNAs in the exosomes produced according to Example 5 of the present invention.
FIG. 18 shows data obtained by performing RNA-seq analysis for RNAs in the exosomes produced according to Example 5 of the present invention.
FIG. 19 shows data obtained by performing qPCR for some RNAs associated with cell proliferation, telomere elongation and anti-senescence in the exosomes produced according to Example 5 of the present invention.
FIG. 20 shows data obtained by performing immunofluorescence analysis for the TERT protein in the exosomes produced according to Example 5 of the present invention.
FIG. 21 shows data obtained by analyzing telomere length changes after cell treatment with the exosomes produced according to Example 5 of the present invention.
FIG. 22 shows data obtained by analyzing telomere length changes after cell treatment with the exosomes produced according to Example 5 of the present invention.
FIG. 23 shows data obtained by performing qFISH analysis of telomeres in cells after treatment of the cells with the exosomes produced according to Example 5 of the present invention.
FIG. 24 shows data obtained by performing Tel-seq analysis for cells after treatment of the cells with the exosomes produced according to Example 5 of the present invention.
FIG. 25 shows data obtained by performing gene ontology analysis for RNA in cells after treatment of the cells with the exosomes produced according to Example 5 of the present invention.
FIG. 26 shows data obtained by performing RNA-seq analysis for RNA in cells after treatment of the cells with the exosomes produced according to Example 5 of the present invention.
FIG. 27 shows data obtained by analyzing the expression of TERT RNA and protein in cells after treatment of the cells with the exosomes produced according to Example 5 of the present invention.
FIG. 28 shows data obtained by analyzing a cell division marker in cells after treatment of the cells with the exosomes produced according to Example 5 of the present invention.
FIG. 29 shows data obtained by analyzing a cell division marker depending on the exosome concentration in cells after treatment of the cells with the exosomes produced according to Example 5 of the present invention.
FIG. 30 shows data obtained by analyzing β-galactosidase activity in cells after treatment of the cells with the exosomes produced according to Example 5 of the present invention.
FIG. 31 shows data obtained by analyzing the telomere length and the expression level of TERT RNA depending on the exosome concentration in mouse ears to which the exosomes produced according to Example 5 of the present invention were applied.
FIG. 32 shows data obtained by performing FISH analysis of telomeres depending on the exosome treatment time in mouse ears to which the exosomes produced according to Example 5 of the present invention were applied.
FIG. 33 shows data obtained by analyzing the RNA and protein expression of cell proliferation-related genes depending on the exosome treatment time in mouse ears to which the exosomes produced according to Example 5 of the present invention were applied.
FIG. 34 shows data obtained by performing immunofluorescence analysis for TERT depending on the exosome treatment time in mouse ears to which the exosomes produced according to Example 5 of the present invention were applied.
FIG. 35 shows data obtained by performing wound healing assay for cells depending on the concentration of the exosomes produced according to Example 5 of the present invention.
FIG. 36 shows data obtained by performing wound healing assay *in vivo* depending on the concentration of the exosomes produced according to Example 5 of the present invention.
FIG. 37 shows data obtained by analyzing tissue penetration after the exosomes produced according to Example 5 of the present invention were applied to the mouse skin.
FIG. 38 shows data obtained by performing telomere-related analysis after cells derived from a progeria patient were treated with the exosomes produced according to Example 5 of the present invention.
FIG. 39 shows data obtained by performing cell proliferation-related analysis after cells derived from a progeria patient were treated with the exosomes produced according to Example 5 of the present invention.
FIG. 40 shows data obtained by analyzing β-galactosidase activity after cells derived from a progeria patient were treated with the exosomes produced according to Example 5 of the present invention.

### [Best Mode]

Hereinafter, the present invention will be described in more detail. However, the present invention may be embodied in various different forms and is not limited by the embodiments described herein, and the scope of the present invention should be defined only by the appended claims.

The terminology used herein is only for the purpose of describing particular embodiments and is not intended to be limiting of the present invention. Singular expressions include plural expressions unless otherwise specified in the context thereof. Throughout the present specification, it is to be understood that when any part is referred to as "comprising" any component, it does not exclude other components, but may further comprise other components, unless otherwise specified.

In addition, the unique name of each gene used under the designation "gene" in the present invention is an officially known gene name, a commonly used name, or the name of a product of the gene, for example, a protein in the case of the gene encoding the protein.

The present inventors previously disclosed in Korean Patent No. 10-1855967 that, when physical stimulation capable of promoting environmental inflow is provided to a mixture of cells and a culture medium and the mixture is provided with the physical stimulation is cultured for a predetermined time, the cells can be reprogrammed, and when cells are treated with exosomes isolated from the mixture, reprogramed cells may be obtained. Here, the direction of reprogramming appears differently depending on the composition of the medium upon application of physical stimulation regardless of the type of cells provided with physical stimulation or the type of cells treated with the exosomes. In a subsequent process of analyzing the effect of the cell reprogramming method, it was confirmed that the expression of the gene group related to telomere elongation was also increased by applying physical stimulation. Through further research thereon, it has been found that, unlike the previously disclosed invention described above, telomeres can be elongated by physical stimulation regardless of not only the type of cells tested but also the composition of medium. Based on this finding, it was possible to conclude that telomere elongation was elongated by the physical stimulation itself rather than the environmental inflow. At this time, it was found that expression of telomere elongation-related factors increased, and secretion of exosomes containing these factors also significantly increased. Based on this finding, a new invention is disclosed.

Exosomes produced according to the method of the present invention contain an RNA or protein of at least one gene selected from among TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin. Most preferably, the exosomes may contain the RNAs or proteins of all the above-described genes.

Here, TERT (telomerase reverse transcriptase) is a subunit having telomerase catalytic activity, and TERF1 (telomeric repeat binding factor 1) and TERF2 (telomeric repeat binding factor 2) recognize the telomere sequence. It is known that DKC1 (dyskerin pseudouridine synthase 1), RFC1 (replication factor C subunit 1), TNKS1BP1 (tankyrase 1 binding protein 1), NBN (Nibrin), HSPA1L (heat shock protein family A member 1 like), PARP1 (poly ADP-ribose polymerase 1), PTGES3 (prostaglandin E synthase 3), SMG6 (Smg6 homolog, Nonsense mediated mRNA decay factor), XRCC5 (X-ray repair cross complementing 5) and XRCC6 (X-ray repair cross complementing 6) are necessary for stabilization and maintenance of telomeres. It has been reported that TERF2IP (TERF2 interacting protein) and RAD50 (RAD50 homolog, double strand break repair protein) inhibit telomere recombination, and PINX1 (PIN2/TERF1-interacting telomerase inhibitor 1) mediate the accumulation of TERF1 and TERT in the nucleolus, help TRF1 bind to telomerase, and inhibit telomerase activity in the S phase. ACD (adrenocortical dysplasia protein homolog), ERCC4 (excision repair cross-complementation group 4) and PRKDC (protein kinase, DNA-activated, catalytic subunit) are necessary to control the telomere length and for telomere protection, and promote telomere amplification during BLM (bloom syndrome protein) DNA synthesis, and telomerase associated protein 1 (TEP1) forms part of the telomerase complex. β-Catenin is a transcriptional regulator that promotes TERT expression. It has been reported that the above-described factors all have effects on the elongation and stabilization of telomeres. The exosomes for elongating telomeres, which contain the above-described gene products, may be used for various effects as described below.

In accordance with the invention, the exosomes are induced by providing ultrasound stimulation directly to cells, e.g. by applying physical stimulation to the medium containing the cells. In particular, the method may be performed by: mixing the cells and the medium and then providing physical stimulation to the mixture; or providing physical stimulation to the cells and then mixing the cells and the medium; or providing physical stimulation to the cells and then mixing the cells and the medium, followed by providing physical stimulation to the mixture; or providing physical stimulation to the medium and then mixing the medium and the cells, followed by providing physical stimulation to the mixture; or providing physical stimulation to each of the cells and the medium and then mixing the cells and the medium; or providing physical stimulation to each of the cells and the medium and then mixing the cells and the medium, followed by providing physical stimulation to the mixture. The physical stimulation may be provided to the cells one or more times or provided using a combination of one or more selected from the above methods, and as the number of times increases, the telomere elongation effect of the exosomes may increase proportionally. When the physical stimulation is provided one or more times as described above, it is preferable to provide a time interval between the provisions so that the cells can recover, and the time interval may be at least 1 day, more preferably at least 2 days.

Providing the ultrasound stimulation directly to the cells is performed at an ultrasound intensity of 0.1 to 3 W/cm² and a frequency of 20 kHz to 20 MHz for a duration of 0.1 seconds to 20 minutes. More preferably, providing the ultrasound stimulation directly to the cells may be performed at an ultrasound intensity of 0.5 to 2 W/cm² and a frequency of 20 kHz to 2 MHz for a duration of 0.1 seconds to 10 minutes.

The exosomes may contain at least 100 ng, preferably at least 150 ng, more preferably at least 150 ng, of total RNA in 1 × 10⁸ exosome particles.

The exosomes may contain at least 0.5 µg, preferably at least 1 µg, more preferably at least 1.2 µg, of total RNA in 1 × 10⁸ exosome particles.

When cells are treated with the exosomes, telomerase activity in the cells may increase. This increase in telomerase activity may lead to elongation of telomere length and promotion of cell division after cell treatment with the exosomes.

When cells are treated with the exosomes, β-galactosidase activity in the cells may decrease. β-galactosidase is an enzyme that catalyzes the hydrolysis of β-galactoside into monosaccharides, and the lysosomal β-galactosidase is overexpressed and accumulated in senescent cells. As a result, it has been reported that the activity of β-galactosidase in senescent cells is high. It was confirmed that, when cells were treated with the exosomes for elongating telomeres of cells according to one embodiment of the present invention, the activity of the enzyme decreased, suggesting that the exosomes have an anti-aging effect.

A composition for elongating telomeres of cells can be obtained, comprising the above-described exosomes.

The exosomes contain gene products of various factors functioning to elongate and stabilize telomeres. The exosomes migrate along the bloodstream and exhibit therapeutic effects by delivering the factors directly to cells. Thus, a composition containing these exosomes may be used for therapeutic purposes in a subject in need of the activities of these genes. For example, the composition may be for use in the treatment or amelioration of diseases known to occur due to abnormal telomere maintenance, such as congenital dysphagia, aplastic anemia, Werner syndrome, Bloom syndrome, Ataxia-telangiectasia, Nijmegen breakage syndrome, Ataxia-telangiectasia-like syndrome, and pulmonary fibrosis. In addition, the composition may be for use in the treatment or amelioration of various diseases and conditions caused by cell senescence and loss, for example, various degenerative diseases such as dementia, ataxia, and degeneration of cartilage and bone, autoimmune diseases such as Crohn's disease and chronic obstructive pulmonary disease, hypertension, metabolic syndrome, diabetes, skin aging, pigmentation abnormalities, hair loss, skin aging, etc. In addition, the composition may be used for rapid regeneration from various injuries such as bums, wounds, injuries, organ failure, organ loss, and ulcers.

Here, the composition may contain the exosomes at a concentration of at least 10² exosomes/ml, preferably 10⁶ to 10¹⁴ exosomes/ml.

The composition may be administered by any one method selected from oral, intravenous, intramuscular, subcutaneous, intradural, and transdermal administration. Preferably, the composition may be administered subcutaneously or transdermally. The composition may contain a pharmaceutically acceptable diluent, preservative, solubilizing agent, emulsifier, adjuvant, buffer and carrier, in addition to an effective amount of the above-described exosomes. Ingredients that may be contained in the composition include buffers such as neutral buffered saline and phosphate buffered saline, carbohydrates such as glucose, mannose, sucrose, dextran and mannitol, amino acids such as polypeptides and glycine, antioxidants, EDTA, chelating reagents such as glutathione, adjuvants such as aluminum hydroxide, and preservatives.

Since the selection of the administration route and the additional ingredients and compositions according to each administration route are known to those skilled in the art, detailed descriptions thereof will be omitted.

It was confirmed that, when the method for producing exosomes for elongating telomeres of cells according to one embodiment of the present invention was applied to various types of cells, it induced secretion of exosomes capable of elongating telomeres from all the types of cells. The cells may be selected from the group consisting of mammalian stem cells, progenitor cells, fibroblasts, keratinocytes, or organ tissue cells. When the cells having elongated telomeres are used for *in vivo* application, the cells may be either autologous, allogeneic or heterologous. When the cells are heterologous, the cells may be of mammalian origin. In order to reduce the likelihood of immune rejection, the cells are preferably allogeneic cells, most preferably autologous cells. The cells may be selected from the group consisting of mammalian stem cells, progenitor cells, fibroblasts, keratinocytes, or organ tissue cells.

The medium may be selected from a culture medium or a differentiation-inducing medium. Here, the term "culture medium" refers to a medium optimized for survival of a specific type of cells while maintaining the uniformity of the cells, which is a medium that is used for uniform cell proliferation. On the other hand, the term "differentiation inducing medium" refers to a medium for inducing differentiation of a specific type of cells into another type of cells having other differentiation potentials or functions.

The culturing of the mixture may be performed for 1 hour to 10 days, preferably 1 to 5 days. The reason therefor is as follows. The reason why this culture time is necessary is as follows. When the above-described physical stimulation is applied to cells, the expression of various genes described later is increased, and in this case, "gene expression" involves transcription, the synthesis and folding of a bioactive substance such as a protein, and the migration of the substance to a necessary position in the cell, and hence it takes time to actually produce exosomes loaded with a bioactive substance having a telomere elongation effect. In addition, it was found that the production of exosomes decreases over time after ultrasound treatment, indicating that an excessively long culture time may not be helpful in the efficiency of exosome production.

The step of isolating the exosomes may be performed using one or more of ultracentrifugation, density gradient separation, filtration, size exclusion chromatography, immunoaffinity separation, precipitation, and microfluidic separation.

The step of isolating the exosomes may comprise steps of: centrifuging the mixture after the culturing to obtain a supernatant; filtering the supernatant through a filter to obtain a filtrate; and concentrating the filtrate.

The expression level of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the isolated exosomes may be higher than that in exosomes secreted from the cells before being subjected to the production method. These genes are known to have functions such as telomere elongation, stabilization and protection, and it was confirmed that, when cells were treated with the exosomes produced by the above-described method, the exosomes promoted telomere elongation and cell growth and also had an anti-aging effect.

When cells are treated with the exosomes, telomerase activity in the cells may increase. This increase in telomerase activity may lead to elongation of telomere length and promotion of cell division after cell treatment with the exosomes.

When cells are treated with the exosomes, β-galactosidase activity in the cells may decrease. As described above, the enzyme β-galactosidase is overexpressed in senescent cells and accumulated in lysosomes, and thus exhibits high activity. It was confirmed that, when cells were treated with exosomes produced by the production method according to one embodiment of the present invention, the activity of the enzyme decreased.

When cells are treated with the isolated exosomes obtained in the method for producing exosomes for elongating telomeres of cells, migration of the cells may increase.

When tissue is treated with the isolated exosomes obtained in the method for producing exosomes for elongating telomeres of cells, regeneration of the tissue may be promoted. As will be described later, the exosomes may increase cell migration and cell proliferation, and may increase expression of a number of genes associated with cell cycle, proliferation and metabolism, in addition to telomere-related genes. This regeneration may target not only physical damage but also chemical damage.

When a wound is treated with the isolated exosomes obtained in the method for producing exosomes for elongating telomeres of cells, healing of the wound may be promoted.

When a scar is treated with the isolated exosomes obtained in the method for producing exosomes for elongating telomeres of cells, healing of the scar may be promoted.

When cells are treated with the isolated exosomes obtained in the method for producing exosomes for elongating telomeres of cells, the exosomes may have an anti-senescence effect on the cells. As described above, it was confirmed that, when cells were treated with exosomes produced by the production method according to one embodiment, β-galactosidase activity in the cells could decrease, and migration and proliferation of the cells could also increase. These effects appears not only in normal cells, but also in progeria patient's cells having problems in terms of telomere elongation, and the above-described exosomes may be for use to delay and prevent senescence or to treat accelerated aging such as progeria.

### [Mode for Invention]

Hereinafter, examples of the present invention will be described in detail so that the present invention can be easily carried out by those skilled in the art. However, the present invention may be embodied in various different forms, and is not limited to the examples described herein.

All cell culture processes in the Examples and Experimental Examples of the present invention were performed at 37°C under 5% CO₂.

### Example 1. Induction of Telomere Elongation in Cells by Direct Ultrasound Stimulation

Ultrasound stimulation was applied to 1×10⁶ cells in 1 ml of medium at an ultrasound intensity of 1 W/cm² and a frequency of 20 KHz for 5 seconds, and the cells were cultured with the same medium in a culture dish for 1 day or more.

### Example 2. (reference) Induction of Telomere Elongation in Cells by Direct Heat Stimulation

1×10⁶ cells in 1 ml of hES medium were placed in a 1.5-ml tube and exposed to 50°C for 5 seconds and then exposed to 0°C for 10 seconds, and the cells were cultured with the same medium in a culture dish for 1 day or more.

### Example 3. (reference) Induction of Telomere Elongation in Cells by Direct Light-Emitting Diode Light Stimulation

Light-emitting diode light stimulation (1 W, 500 Hz/sec) with a wavelength of 808 nm was applied directly to 1×10⁶ cells in 1 ml of hES medium for 5 seconds, and the cells were cultured with the same medium in a culture dish for 1 day or more.

### Example 4. (reference) Induction of Telomere Elongation in Cells by indirect Ultrasound Stimulation

hES medium was treated with ultrasound at 0, 3, 5 and 10 W/cm² for 10 minutes and then mixed with non-ultrasound-treated cells, followed by 1 day or more of culture.

### Example 5. Production of Exosomes Capable of inducing Cell Telomere Elongation by Ultrasound Stimulation

Ultrasound stimulation was applied to 1×10⁶ cells in 1 ml of medium at an ultrasound intensity of 1 W/cm² and a frequency of 20 KHz for 5 seconds, and the cells were cultured with the same medium in a culture dish for 1 to 2 days. Next, the conditioned medium was collected and centrifuged at 3000 rpm for 5 minutes to remove cell debris or dead cells, and only the supernatant was collected and filtered through a 0.2-µm filter. The filtrate containing only components having a size of 0.2 µm or less was collected, placed in a 100-kDa filter, and centrifuged at 10,000xg for 30 minutes to remove components having a molecular weight of 100 kDa or less, thus obtaining concentrated exosomes. PBS was added to the 100-kDa filter containing the concentrated exosomes and washed at 10000xg for 5 minutes, and this addition and washing process was repeated twice to remove the medium component from the exosome concentrate, thus obtaining exosomes.

### Example 6. Elongation of Telomeres in Cells Using Exosomes Capable of Inducing Cell Telomere Elongation

The exosomes (reprosome) produced according to Example 5 were added to medium at a concentration of 1 × 10⁹ particles/ml, and cells were cultured in the medium.

### Experimental Example 1. Analysis of Telomere Elongation Effect Depending on Type of Cells

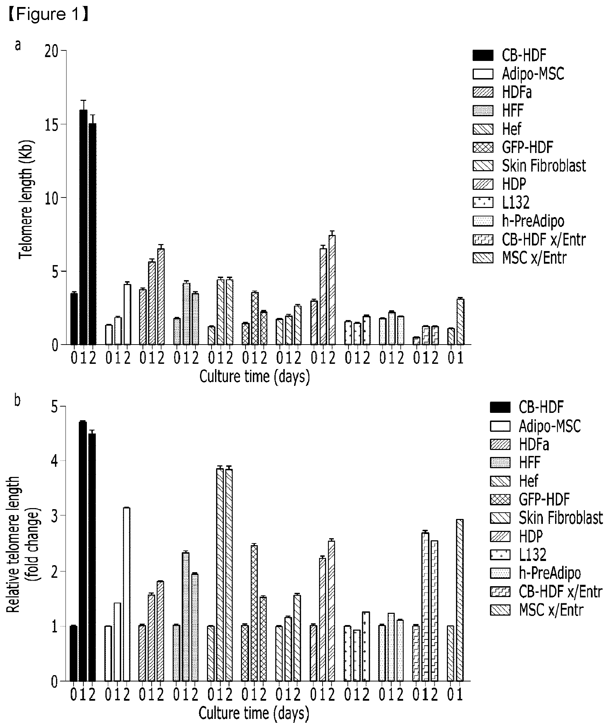

In order to compare the effect of Example 1 depending on the type of cells, ultrasound stimulation was added to each of the following types of 1×10⁶ cells in 1 ml of DMEM medium (fibroblast culture medium) according to the method of Example 1: CB-HDFs (CellBio), Adipo-MSC cells (professor Hwang Dong-Yeon Lab.), HDFa cells (Invitrogen), HFF cells (Cha University), Hef cells (Cha University), GFP-HDF cells (GFP-HNDFs, Angio-proteomie), skin fibroblasts (obtained from a 6o-year-old stroke patient sample in compliance with the IRB-approved protocol), HDP cells (CellBio), L132 cells (ATCC), h-PreAdipo cells (ATCC), CB-HDF x/Entr cells, and MSC x/Entr cells (here, CB-HDF x/Entr and MSC x/Entr cells are pluripotent cells derived from CB-HDF and MSC cells, respectively, in human embryonic stem cell culture medium by application of a method of inducing pluripotent cells through ultrasound treatment in the study conducted by the present inventor (Lee et al., An ultra-effective method of generating extramultipotent cells from human fibroblasts by ultrasound, Biomaterials, 2017.)). Then, the cells were cultured for 0, 1 and 2 days, and the telomere lengths in the cells were analyzed according to a qPCR method using a kit (Absolute Human Telomere Length Quantification qPCR Assay Kit, Cat No. 8918, ScienCell^{™}). As a result, as shown in FIG. 1 and Table 1 below, it was confirmed that telomeres in all the types of cells were elongated.

**[Table 1]**

| Type of cell | Change (fold) in telomere length relative to control | |
|---|---|---|
| | Absolute value (kb) | Relative value (fold) |
| CB-HDF | 12.04 | 4.47 |
| MSC | 1.65 | 2.28 |
| HDFa | 2.36 | 1.79 |
| HFF | 2.00 | 2.14 |
| Hef | 3.22 | 3.80 |
| GFP-HDF | 2.36 | 1.79 |
| Skin Fibroblast | 0.57 | 1.34 |
| HDP | 4.05 | 2.40 |
| L132 | 1.30 | 1.54 |
| h-PreAdipo | 3.15 | 2.44 |
| CB-HDF x/Entr | 0.85 | 1.26 |
| MSC x/Entr | 1.82 | 2.93 |

### Experimental Example 2. Analysis of Telomere Elongation Effect Depending on Kind of Physical Stimulation

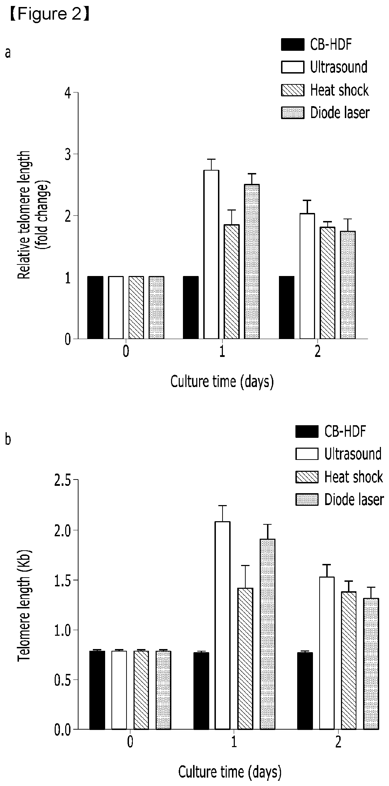

To analyze the effect of physical stimulation on telomere analysis depending on the kind of physical stimulation, each physical stimulation was added to CB-HDFs in human embryonic stem cell culture medium according to the methods of Examples 1 to 3, and then the cells were cultured for 0, 1 and 2 days, and the telomere length in the cells was analyzed according to a qPCR method. As a result, as shown in FIG. 2, it was confirmed that ultrasound, heat and light stimuli all elongated telomeres.

### Experimental Example 3. (reference) Analysis of Telomere Elongation Effect of Indirect Ultrasound Stimulation

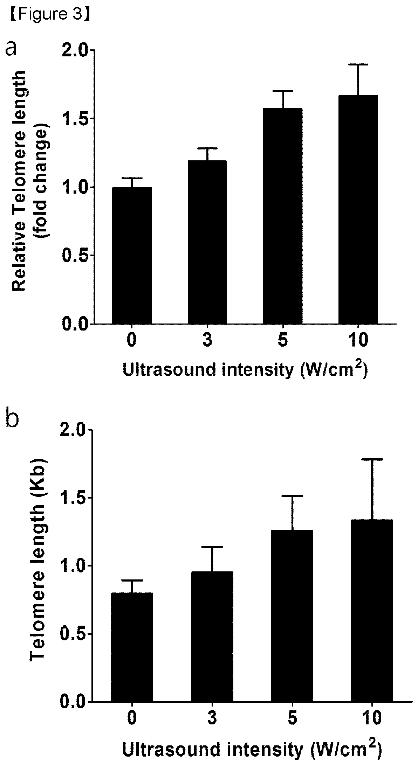

In order to examine whether telomeres are elongated even by indirect physical stimulation, not by direct physical stimulation, indirect ultrasound stimulation was applied to CB-HDFs according to the method of Example 2, and then the cells were cultured for 1 day, and the telomere length in the cells were analyzed according to a qPCR method. As a result, as shown in FIG. 3, it was confirmed that the effect on telomere elongation was great in proportion to the intensity of the ultrasound used.

### Experimental Example 4. Analysis of Telomere Elongation Effect After Ultrasound Treatment Depending on Medium Composition

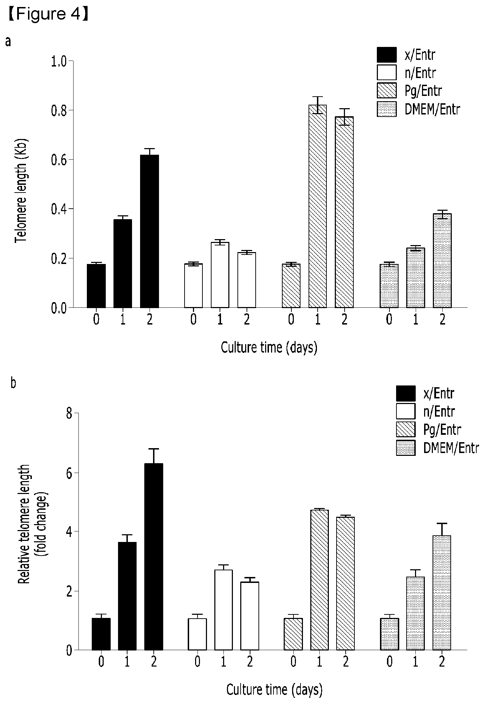

In order to compare the effect of Example 1 depending on the composition of medium, ultrasound was applied to 1×10⁶ CB-HDFs in 1 ml of each of human embryonic stem cell culture medium, neural stem cell culture medium, primary germ cell culture medium and DMEM medium according to the method of Example 1, and then the cells were cultured for 0, 1 and 2 days, and the telomere length in the cells was analyzed according to a qPCR method. As a result, as shown in FIG.4, it was confirmed that telomeres were elongated regardless of the composition of medium.

### Experimental Example 5. Analysis of Telomere Elongation Effect Depending on Number of Ultrasound Treatments

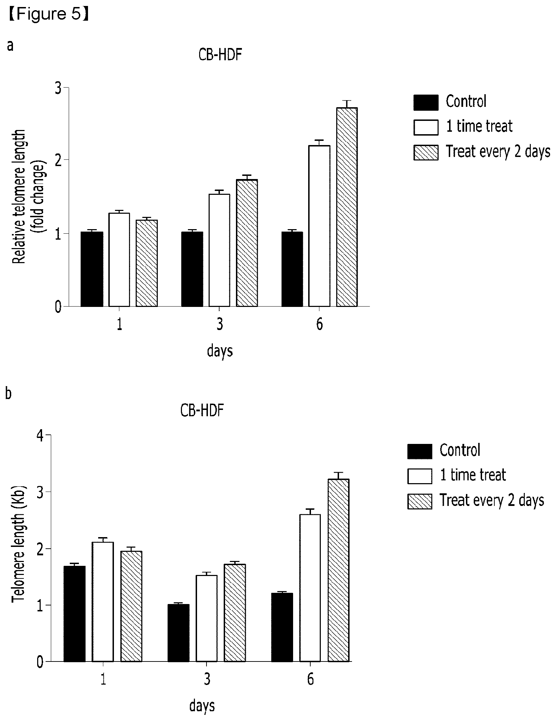

In order to examine whether a difference appears when the ultrasound according to Example 1 is repeated, ultrasound stimulation was applied to 1×10⁶ CB-HDFs in 1 ml of DMEM medium according to the method of Example 1, and the cells were cultured for 1, 3 and 6 days. In this case, the ultrasound stimulation was applied one time or applied every 2 days. The telomere length in the cells was analyzed according to a qPCR method. As a result, as shown in FIG. 5, it was confirmed that the telomere length increased regardless of the number of ultrasound treatments, and as the number of ultrasound treatments increased, the increment in the telomere length compared to the control not treated with ultrasound increased.

### Experimental Example 6. Analysis of TERT Gene Expression After Ultrasound Treatment Depending on Type of Cells

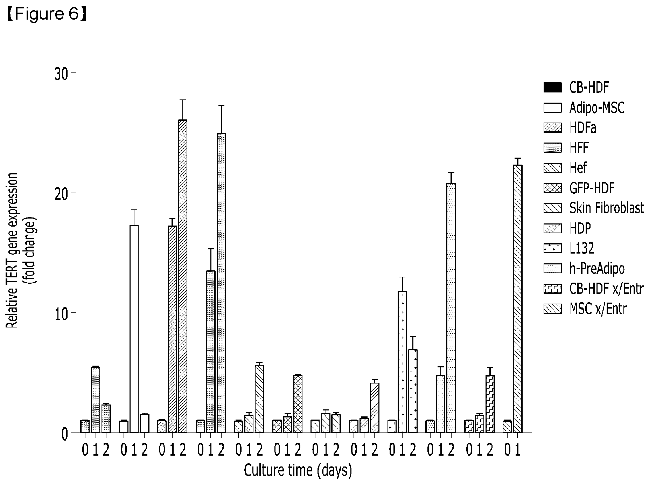

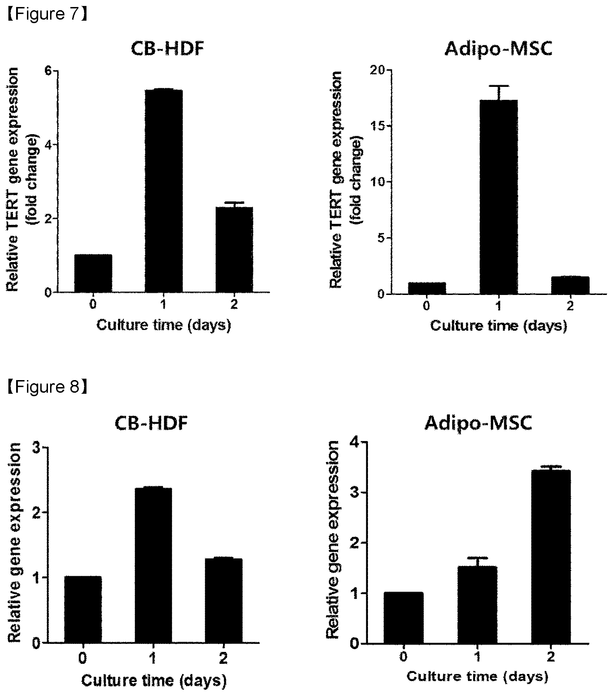

In order to examine whether the method of Example 1 shows a difference in the expression level of TERT gene depending on the type of cells, ultrasound stimulation was applied to each of the following types of 1×10⁶ cells according to the method of Example 1: CB-HDFs, Adipo-MSC cells, HDF cells, HFF cells, Hef cells, GFP-HDF cells, skin fibroblasts, HDP cells, L132 cells, h-PreAdipo cells, CB-HDF x/Entr cells and MSC x/Entr cells. Then, the cells were cultured for 0, 1 and 2 days, and changes in TERT gene expression in the cells were analyzed according to a qPCR method. As a result, as shown in FIG. 6 and Table 2 below, it was confirmed that the expression levels of TERT in all the types of cells increased. In addition, as shown in FIG. 7, it was shown that the expression levels of TERT in CB-HDFs and Adipo-MSC cells significantly increased on day 1 of culture after ultrasound treatment and then decreased again on day 2. That is, since continuous expression of TERT has a risk of development of cancer, etc., it is expected that temporary expression of TERT may ameliorate aging-related conditions caused by telomere shortening while reducing this risk.

**[Table 2]**

| Type of cell | Change (fold) in TERT gene expression relative to control |
|---|---|
| CB-HDF | 5.4 |
| MSC | 19.46 |
| HDFa | 21.61 |
| HFF | 16.81 |
| Hef | 3.50 |
| GFP-HDF | 3.05 |
| Skin Fibroblast | 1.53 |
| HDP | 2.66 |
| L132 | 6.88 |
| h-PreAdipo | 11.92 |
| CB-HDF x/Entr | 3.08 |
| MSC x/Entr | 22.20 |

### Experimental Example 1. Analysis of Change in Intracellular β-Catenin Gene Expression by Ultrasound Treatment

In order to examine how the telomere elongation method according to Example 1 affects the β-catenin gene which is a transcriptional activator of TERT, ultrasound stimulation was applied to 1×10⁶ CB-HDFs in 1 ml of DMEM medium and 1×10⁶ Adipo-MSC cells in 1 ml of DMEM medium according to the method of Example 1, and then the cells were cultured for 0, 1 and 2 days, and changes in β-catenin gene expression in the cells were analyzed according to a qPCR method. As a result, as shown in FIG. 8, the expression levels of β-catenin increased in the two types of cells tested all increased.

### Experimental Example 8. Analysis of Change in Intracellular Telomerase Activity by Ultrasound Treatment

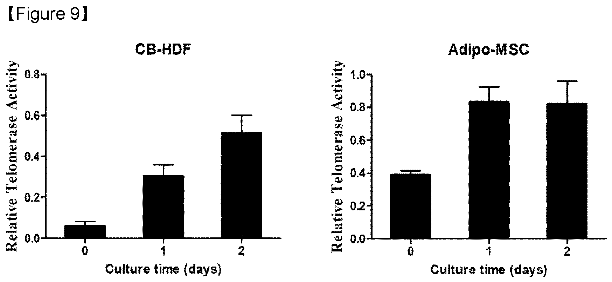

In order to examine how the telomere elongation method according to Example 1 affects telomerase activity, ultrasound stimulation was applied to 1×10⁶ CB-HDFs in 1 ml of DMEM medium and 1×10⁶ Adipo-MSC cells in 1 ml of DMEM medium according to the method of Example 1, and then the cells were cultured for 0, 1 and 2 days, and telomerase activities in the cells were analyzed by Sciencell's Telomerase Activity Quantification qPCR kit (TAQ). As a result, as shown in FIG. 9, it was confirmed that telomerase activities in the two types of cells tested all increased.

### Experimental Example 9. FISH Analysis of Telomeres in Ultrasound-Treated Cells

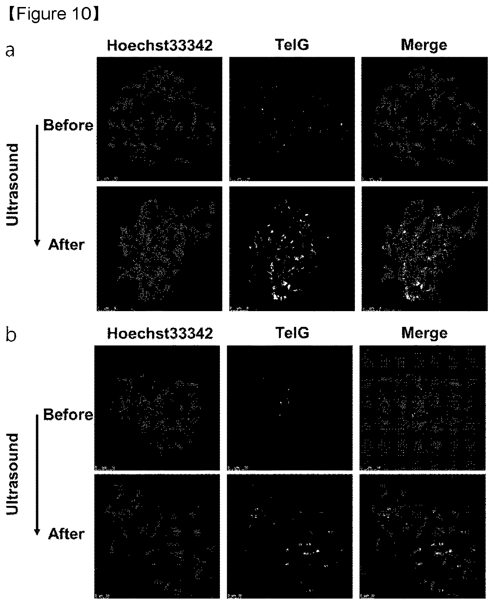

In order to further confirm that telomere elongation is induced by the telomere elongation method according to Example 1, ultrasound stimulation was applied to 1×10⁶ CB-HDFs in 1 ml of DMEM medium and 1×10⁶ Adipo-MSC cells in 1 ml of DMEM medium according to the method of Example 1, and then the cells were cultured for 1 day. Then, fluorescence *in situ* hybridization (FISH) analysis was performed using a TelG telomere probe (TTAGGGTTAGGGTTAGGG), and fluorescent signals were analyzed with a confocal microscope. At this time, the cells were counterstained with Hoechst 33342. As a result, as shown in FIG. 10, it was confirmed that the amounts of telomeres in the two types of cells tested all increased.

### Experimental Example 10. Cell Immunofluorescence Staining for Ki67 and TERT in Ultrasound-Treated Cells

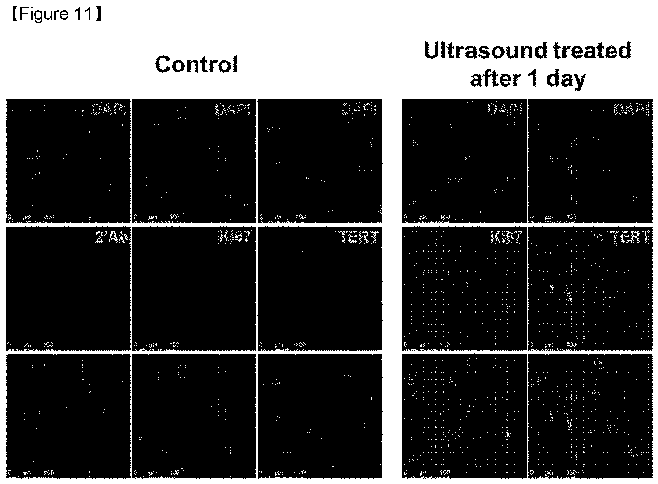

In order to confirm whether TERT gene expression induced by the telomere extension method according to Example 1 leads to an increase in protein expression, ultrasound stimulation was applied to 1×10⁶ CB-HDFs in 1 ml of DMEM medium according to the method of Example 1, and the cells were cultured for 1 day, and then fluorescence-stained using anti-Ki67 and anti-TERT antibodies and observed with a confocal microscope. As a result, as shown in FIG. 11, it was confirmed that, when the cells were treated with ultrasound, expression of the telomere-related TERT protein increased and expression of the cell division marker Ki67 protein also increased.

### Experimental Example 11. Analysis of Senescence-Related β-Galactosidase Activity

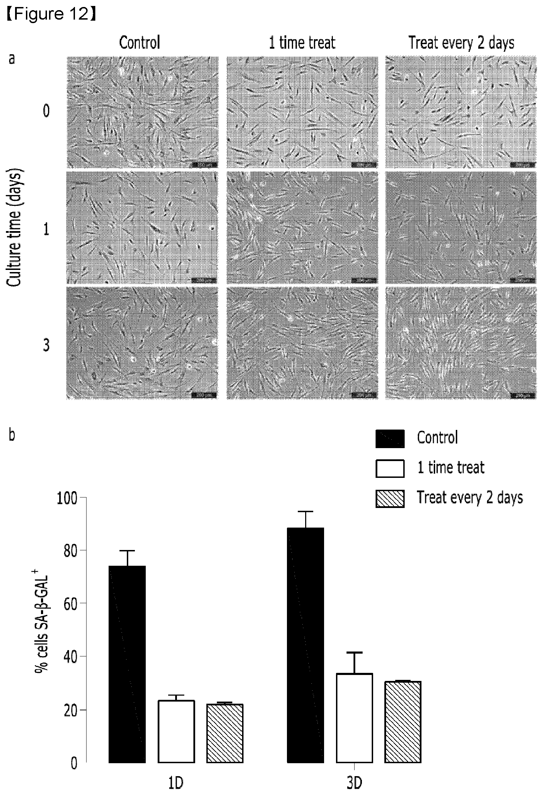

In order to examine how the above-described changes caused by the telomere elongation method according to Example 1 affects cell senescence, ultrasound stimulation was applied to 1×10⁶ CB-HDFs in 1 ml of DMEM medium according to the method of Example 1, and the cells were cultured for 1 day and 3 days. Then, a test for β-galactosidase activity known to have a correlation with intracellular senescence was performed using X-gal, followed by analysis with a phase contrast microscope. At this time, the ultrasound treatment was performed one time or performed every 2 days. As a result, as shown in FIG. 12, it could be confirmed that β-galactosidase activity decreased, suggesting that the method for elongating telomeres according to the present invention has an anti-aging effect.

### Experimental Example 12. Analysis of Exosome Production Efficiency of Production Method Using Ultrasound Treatment and Analysis of Components in Exosomes

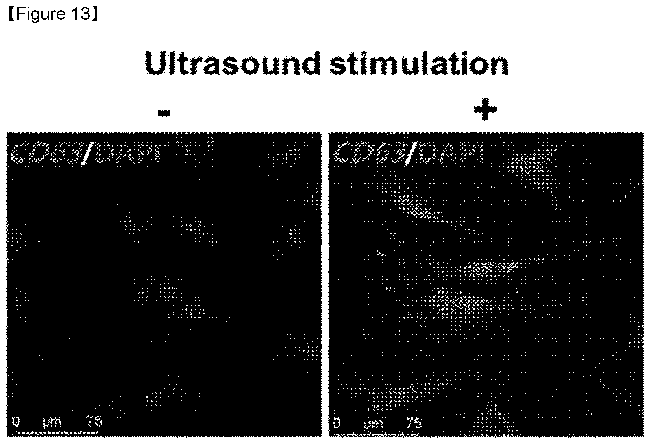

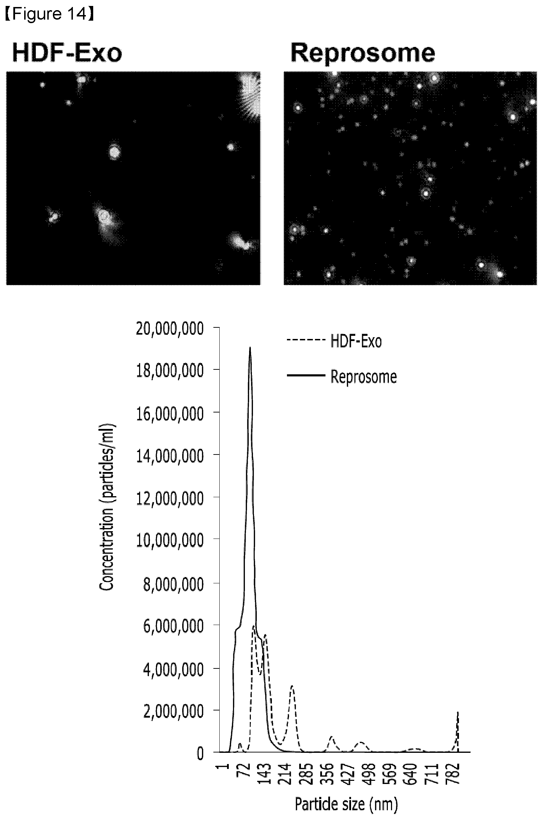

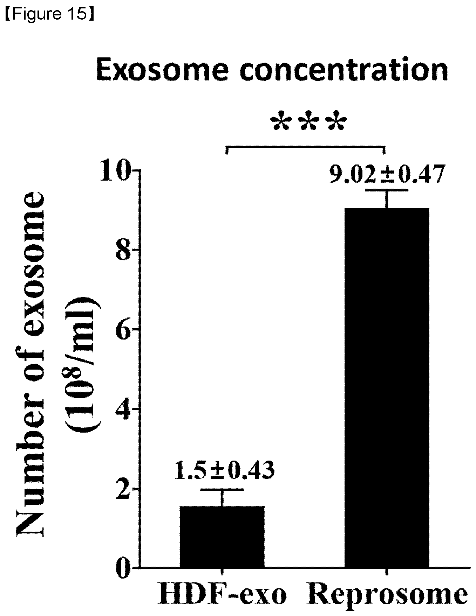

Exosomes were produced according to the method of Example 5, and the exosome production efficiency was analyzed. First, expression of an exosome marker (CD63) in HDFs 24 hours after ultrasound treatment was analyzed immunofluorescence staining (at this time, counter staining was performed using the nuclear staining dye DAPI). As a result, it could be confirmed that the expression of the exosome marker significantly increased in the exosome-treated cells compared to the non-exosome-treated group (FIG. 13). Whether expression of the marker actually leads to exosome secretion was analyzed using a Nanosight LM10 microscope, and as a result, it could be confirmed that, in the ultrasound-treated group, a large amount of exosomes having a diameter of particularly 30 to 200 nm were secreted, and particularly, the total number of exosomes was 9.02 ± 0.47 × 10₈ exosomes/ml, which was at least 5 times larger than that in the non-ultrasound-treated group (1.5 ± 0.43 × 10⁸ exosomes/ml) (FIGS. 14 and 15).

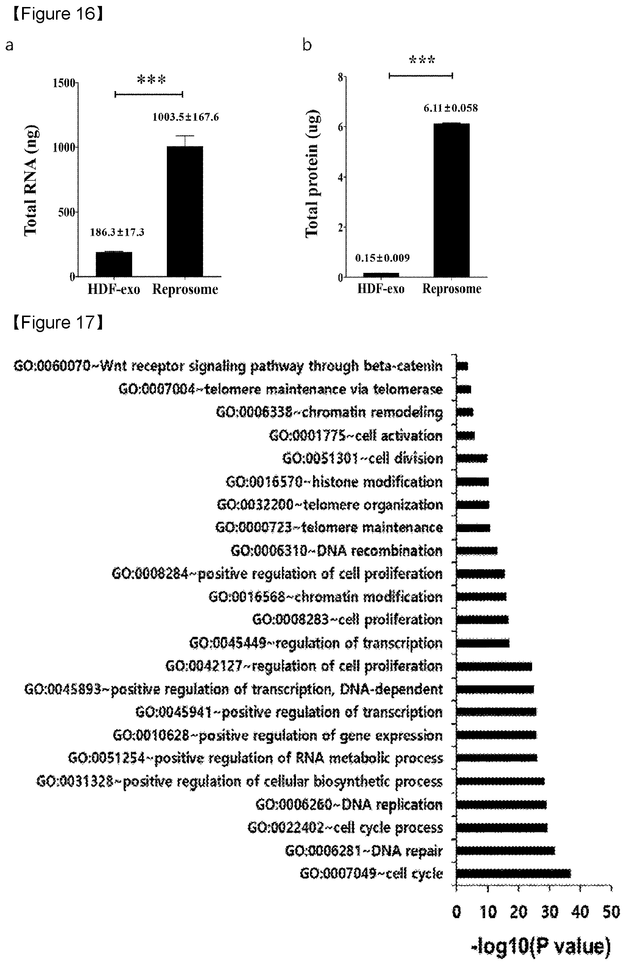

Next, analysis was performed as to whether there were changes in the total amounts of RNA and protein in the exosomes whose secretion was induced by ultrasound treatment as described in Example 5. Total RNA was extracted from the exosomes using TRIzol (RNAi, Takara, Otsu, Japan), and then quantified by Agilent 2100 Bioanalyzer (Agilent, Santa Clara, CA, USA). As a result, it could be confirmed that the amount of total RNA in the exosomes secreted from 1 × 10⁶ cells was 1003.5 ± 167.6 ng when ultrasound treatment performed was performed as described in Example 5, which was at least 5 times larger than when ultrasound treatment was not performed (186.3 ± 17.3 ng). At this time, the amount of total RNA in the same number of exosomes (1 × 10⁸ exosomes) was 222.4 ± 37.1 ng in the case of ultrasound treatment, which was similar to that in the case of non-ultrasound treatment (241.5 ± 22.5 ng) (FIG. 16 and Table 3). As a result of quantifying the total protein by the BCA method, it could be confirmed that the amount of the total protein in the exosomes secreted from 1 × 10⁶ cells was 6.11±0.058 µg when ultrasound treatment was performed as described in Example 5, which was about 40 times larger than when ultrasound treatment was not performed (0.15 ± 0.009 µg). At this time, the amount of total protein in the same number of exosomes (1 × 10⁸ exosomes) was 1.35 ± 0.013 µg in the case of ultrasound treatment, which was at least 5 times larger than that in the case of non-ultrasound treatment (0.20 ± 0.012 µg) (FIG. 16 and Table 3).

**[Table 3]**

| | Total RNA (ng) | | Total protein (ug) | |
|---|---|---|---|---|
| Sample name | HDF-exo | Reprosome | HDF-exo | Reprosome |
| Average | 241.5356 | 222.4102 | 0.197332 | 1.354832 |
| SEM | 22.4809 | 37.14242 | 0.011881 | 0.012999 |

### Experimental Example 13. Analysis of RNA and Protein in Exosomes Secreted from Cells by Ultrasound Treatment

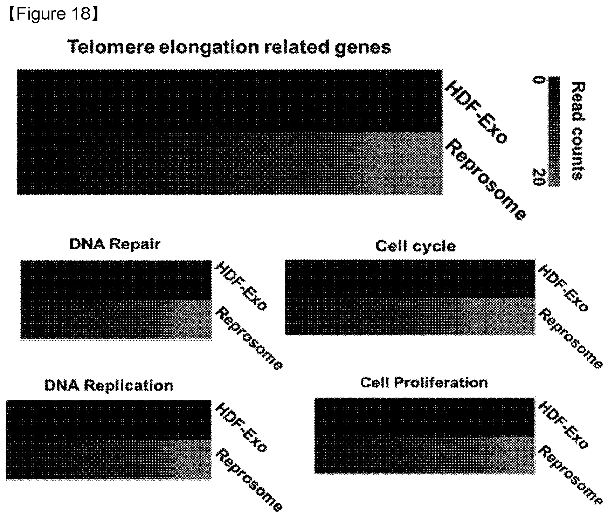

In order to identify RNAs in the exosomes produced according to Example 5, RNA-seq was performed and gene ontology analysis was performed. As a result, it was confirmed that a number of genes associated with telomere maintenance and protection, cell cycle and proliferation, and DNA repair and amplification were present in the exosomes (FIGS. 17 and 18). Particularly, as shown in Table 4 below, it was confirmed that expression of telomerase activity-related genes and genes associated with telomere maintenance and protection in the exosomes secreted from the cells of the experimental group increased.

**[Table 4]**

| | Genes | Read count | |
|---|---|---|---|
| | | HDF.Exo | x.Exo |
| Telomerase activity-related genes | TNKS1BP1 | 0 | 17 |
| | PINX1 | 0 | 15 |
| | TERF1 | 0 | 7 |
| | RAD50 | 0 | 6 |
| | RFC1 | 0 | 5 |
| | TERF2IP | 0 | 5 |
| | DKC1 | 0 | 3 |
| | TERF2 | 0 | 3 |
| | TERT | 0 | 3 |
| Genes associated with telomere maintenance and protection | TEP1 | 0 | 94 |
| | PRKDC | 0 | 33 |
| | ERCC4 | 0 | 23 |
| | XRCC5 | 0 | 19 |
| | XRCC6 | 0 | 18 |
| | TNKS1BP1 | 0 | 17 |
| | PINX1 | 0 | 15 |
| | BLM | 0 | 11 |
| | SMG6 | 0 | 11 |
| | PTGES3 | 0 | 10 |
| | PARP1 | 0 | 7 |
| | TERF1 | 0 | 7 |
| | RAD50 | 0 | 6 |
| | RFC1 | 0 | 5 |
| | TERF2IP | 0 | 5 |
| | HSPA1L | 0 | 4 |
| | NBN | 0 | 4 |
| | ACD | 0 | 3 |
| | DKC1 | 0 | 3 |
| | TERF2 | 0 | 3 |
| | TERT | 0 | 3 |

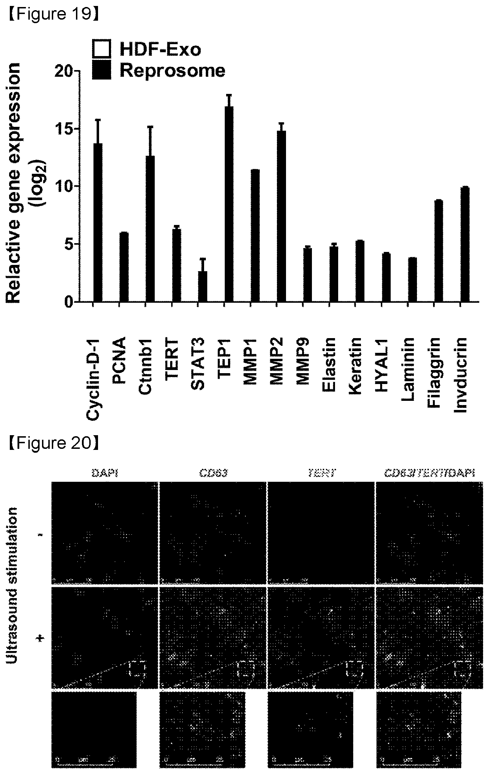

In addition, as a result of RNAs associated with cell proliferation, telomere lengthening and anti-senescence in the exosomes by qPCR, it could be confirmed that RNAs of cell proliferation-related cyclin-D1 and PCNA, telomere lengthening-related Ctnnb1, TERT and STAT3, and anti-senescence-related TEP1, MMP1, MMP2, MMP9, elastin, keratin, HYAL1, laminin, filaggrin and invducrin, significantly increased compared to those in the control group in which they were hardly detected (FIG. 19).

To confirm TERT protein in the exosomes secreted by ultrasound stimulation, immunofluorescence staining for the exosome marker and the TERT protein was performed. As a result, it was possible to confirm a number of portions where the two signals overlap (FIG. 20). Taking these results with the results of analysis of RNAs in the exosomes, it appears that the exosomes produced according to Example 5 contain large amounts of RNAs and proteins associated with telomere lengthening, cell proliferation and anti-senescence.

### Experimental Example 14. Analysis of Telomeres in Cells Treated with Exosomes Secreted by Ultrasound Treatment

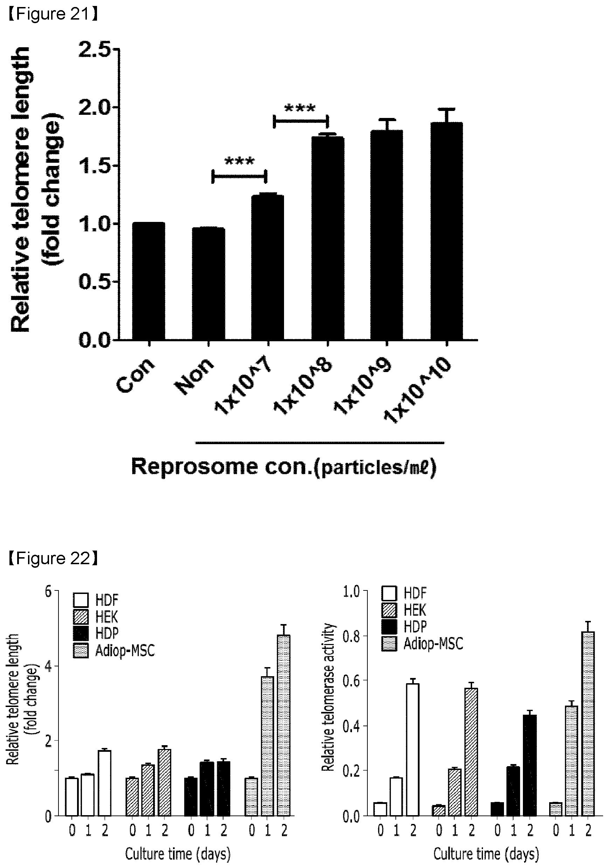

In order to examine how the exosomes produced according to Example 5 affect telomere elongation in cells, the cells were cultured in a culture medium containing the exosomes, and after 2 days, the telomere length was analyzed according to a qPCR method. As a result, it was confirmed that, as the concentrations of the exosomes increased, the telomere length significantly increased in the exosome-treated cells compared to the cells (con) before exosome treatment and the group (non) cultured for 2 days without exosome treatment (FIG. 21).

Next, in order to examine whether the telomeres elongation effect of the exosome appears differently depending on the type of cells, each type of HDF, HEK, HDP and Adipo-MSC cells was cultured for 0, 1 and 2 days in a culture medium containing the exosomes at a concentration of 1 × 10⁹ exosomes/ml according to the method of Example 6, and the telomere length and telomerase activity in each type of cells were analyzed. As a result, it could be confirmed that, in all the four types of cells tested, telomeres were significantly elongated and telomerase activity was also high (FIG. 22). Taken together, it can be confirmed that the exosomes produced according to the production method of the present invention can have a telomere elongation effect regardless of the type of cells when the cells are treated with the exosomes.

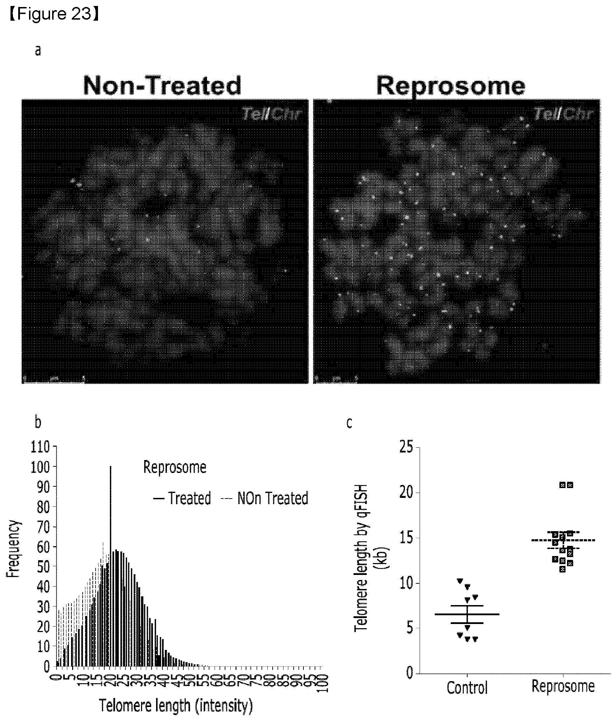

To further verify telomere elongation, staining with a Cy3-labeled PNA telomere probe (complementary to the telomere repeat sequence TTAGGG) and Hoechst33342 was performed. At this time, as a pretreatment before the experiment, medium was treated with Colcemid for 48 hours to fix the cells to the division metaphase in which intranuclear chromosomes are generated. Then, the chromosome in the cell nucleus was mounted onto slide glass using a low osmotic pressure medium and a fixative, and then a solution containing hybridization buffer and a PNA probe (pre-warmed to 90°C) was added to the chromosome, and then the cells were incubated at 85°C for 10 minutes, and then cooled slowly at room temperature for 1 hour. Then, the cells were washed with 2xSSC buffer and stained with the nuclear staining reagent Hoechst 33342, followed by mounting. Then, expression of telomere fluorescence compared to the chromosome was analyzed with a confocal laser microscope at 1,000x magnification. As a result, as shown in FIG. 23a, the frequency at which Cy3 fluorescence more strongly appeared was higher in the cell group cultured in the culture medium containing the exosomes at a concentration of 1 × 10⁹ exosomes/ml for 2 days than in the control group not treated with the exosomes. Next, the difference in telomeres fluorescence intensity compared to the chromosome (Hoechst33342) was compared between the experimental group and the control group (FIG. 23b), and qFISH analysis of telomeres was performed to analyze the telomere length from the average of the analyzed values (FIG. 23c). As a result, it could be confirmed that the telomeres in the exosome-treated cells were significantly elongated.

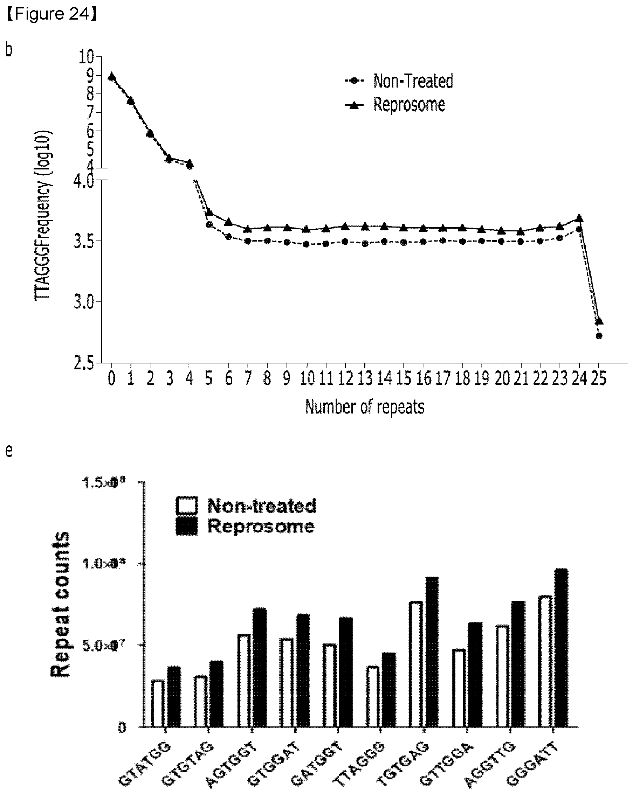

Next, the frequency of repeat sequences at telomere ends was analyzed through whole genome sequencing. First, as a result of analyzing the frequency of the TTAGGG sequence, it could be confirmed that the TTAGGG sequence appeared more frequently in the cell group cultured in the culture medium containing the exosomes at a concentration of 1 × 10⁹ exosomes/ml for 2 days than in the control group not treated with the exosomes (FIG. 24a). Telomeres having a complex structure may appear in a variety of sequences during sequencing, and as a result of additionally analyzing the frequency of the possible repeat sequences, it was confirmed that all the possible sequences were highly frequent in the experimental group (exosome-treated group) (FIG. 24b and Table 5).

**[Table 5]**

| Sample | Repeat sequence | Total Repeat Counts |
|---|---|---|
| Non-Treated | GTATGG | 28595288 |
| | GTGTAG | 31109759 |
| | AGTGGT | 56157875 |
| | GTGGAT | 53677254 |
| | GATGGT | 50041670 |
| | TTAGGG | 36690395 |
| | TGTGAG | 76312707 |
| | GTTGGA | 47035230 |
| | AGGTTG | 61574758 |
| | GGGATT | 79803631 |
| Reprosome | GTATGG | 36398934 |
| | GTGTAG | 40032083 |
| | AGTGGT | 72108868 |
| | GTGGAT | 68187857 |
| | GATGGT | 66658272 |
| | TTAGGG | 45160936 |
| | TGTGAG | 91292791 |
| | GTTGGA | 63576514 |
| | AGGTTG | 76579716 |
| | GGGATT | 95902062 |

### Experimental Example 15. Analysis of Gene Expression in Cells Treated with Exosomes Secreted by Ultrasound Treatment

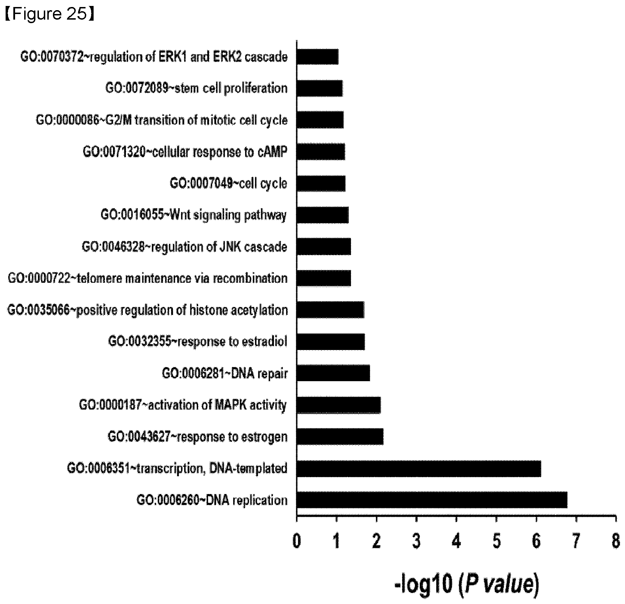

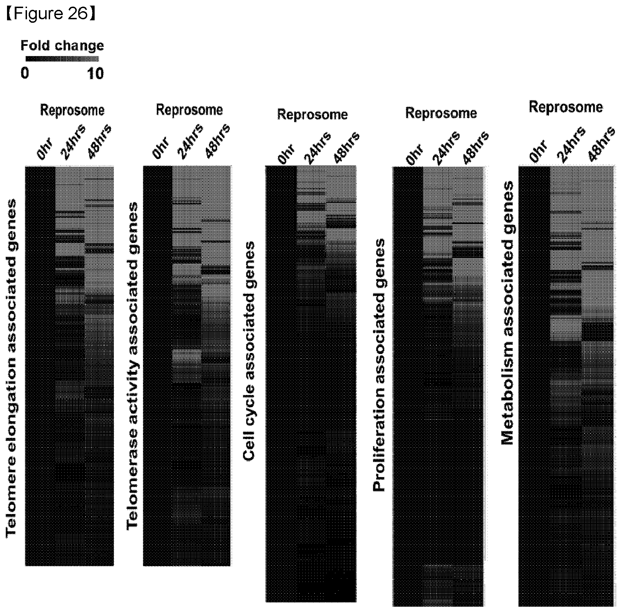

To identify RNAs in the cells treated with the exosomes according to the method of Example 6, RNA-seq was performed and gene ontology analysis was performed. As a result, it was confirmed that a number of genes associated with telomere elongation, telomerase activity, cell cycle and proliferation, and metabolism were present in the cells (FIGS. 25 and 26). Particularly, as shown in Table 3, it was confirmed that expression of telomerase activity-related genes and genes associated with telomere maintenance and protection in the exosomes secreted from the cells of the experimental group increased.

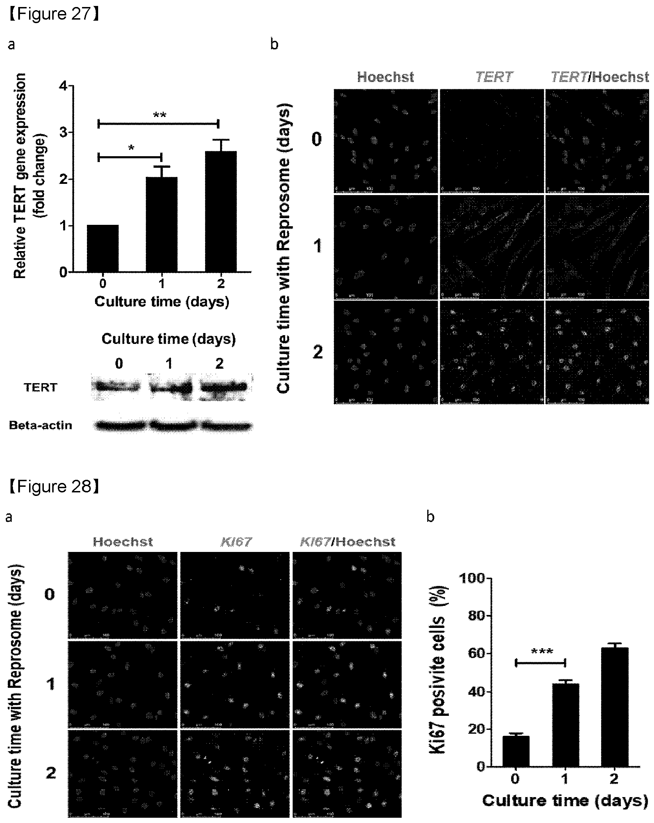

Next, in order to confirm the expression of TERT in the cells treated with the exosomes according to Example 6, qPCR and immunofluorescence staining were performed after 0, 1 and 2 days exosome treatment. As a result, it was confirmed that the amounts of TERT RNA and protein increased as the exosome treatment time increased (FIG. 27).

### Experimental Example 16. Cell Immunofluorescence Staining for Ki67 in Cells Treated with Ultrasound-Induced Exosomes

In order to determine how the change in gene expression in cells treated with the ultrasound-induced exosomes affects cell proliferation, HDFs were cultured for 0, 1 and 2 days in a medium containing the exosomes according to the method of Example 6, and then fluorescence-stained using anti-Ki67 antibody, followed by analysis with a confocal microscope (at this time, the cells were counter-stained with Hoechst). As a result, as shown in FIG. 28, it was confirmed that expression of the cell division marker Ki67 protein increased as the time of co-culture with the exosomes increased.

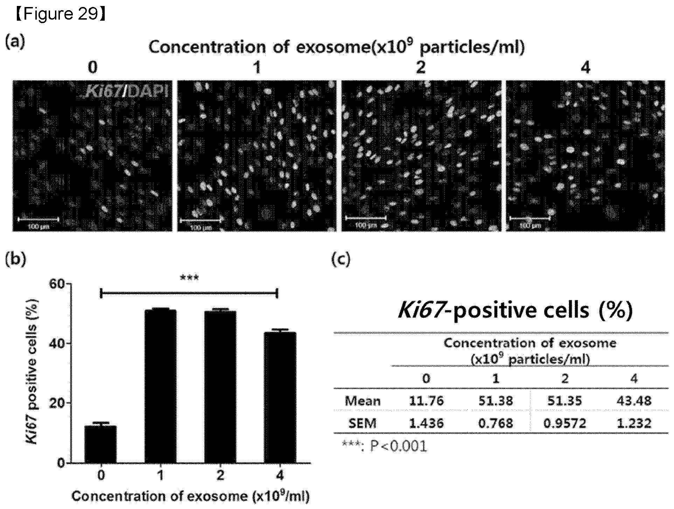

In addition, in order to examine how the concentration of the exosome affects cell proliferation, HDFs were cultured for 2 days in a medium containing the exosomes at a concentration of each of 0, 1, 2 and 4 (×10⁹ exosome particles/ml), then fluorescence-stained using anti-Ki67 antibody, followed by analysis with a confocal microscope (at this time, the cells were counter-stained with Hoechst). As a result, as shown in FIG. 29 and Table 6 below, it was confirmed that, in the exosome concentration range tested, the number of cells expressing the cell division marker Ki67 protein in the exosome-treated cells significantly increased compared to that in the control group.

**[Table 6]**

| % Ki67-positive cells | Concentration of exosomes (×10⁹ particles/ml) | | | |
|---|---|---|---|---|
| | 0 | 1 | 2 | 4 |
| Mean | 11.76 | 51.38 | 51.35 | 43.48 |
| SEM | 1.436 | 0.768 | 0.9572 | 1.232 |

### Experimental Example 17. Analysis of Cell Senescence-Related β-Galactosidase Activity in Cells Treated with Ultrasound-Induced Exosomes

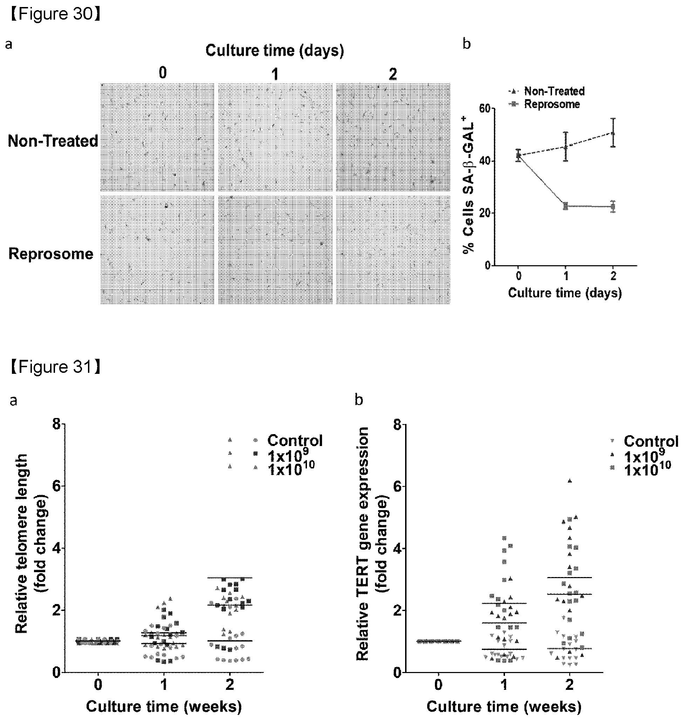

In order to examine how the change in gene expression in cells treated with the ultrasound-induced exosomes affects cell senescence, HDFs were cultured for 0, 1 and 2 days in a medium containing the exosomes according to the method of Example 6, a test for β-galactosidase activity known to have a correlation with intracellular senescence was performed using X-gal, followed by analysis with a phase contrast microscope. As a result, as shown in FIG. 30, it could be confirmed that β-galactosidase in the cells was decreased, suggesting that the exosomes produced according to the present invention may have an anti-senescence effect.

### Experimental Example 18. Analysis of Telomere Elongation Effect of In Vivo Application of Ultrasound-Induced Exosomes and Analysis of Change in TERT Gene Expression

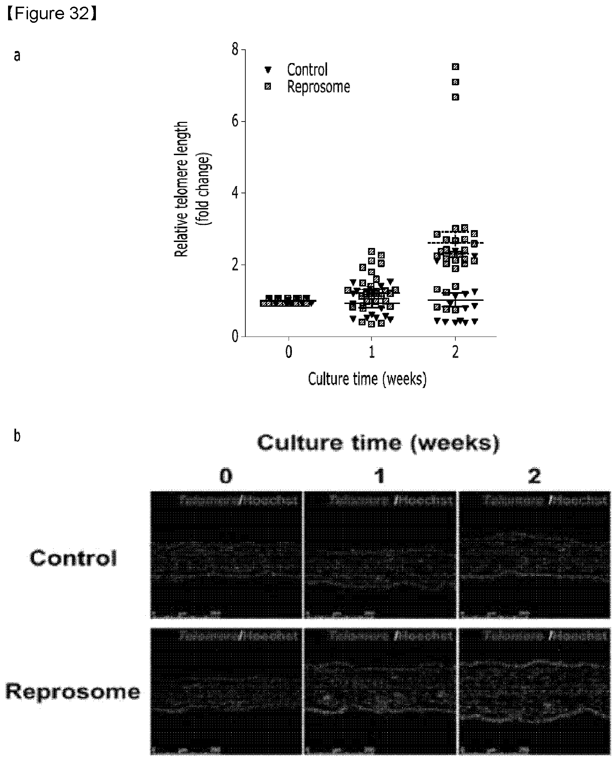

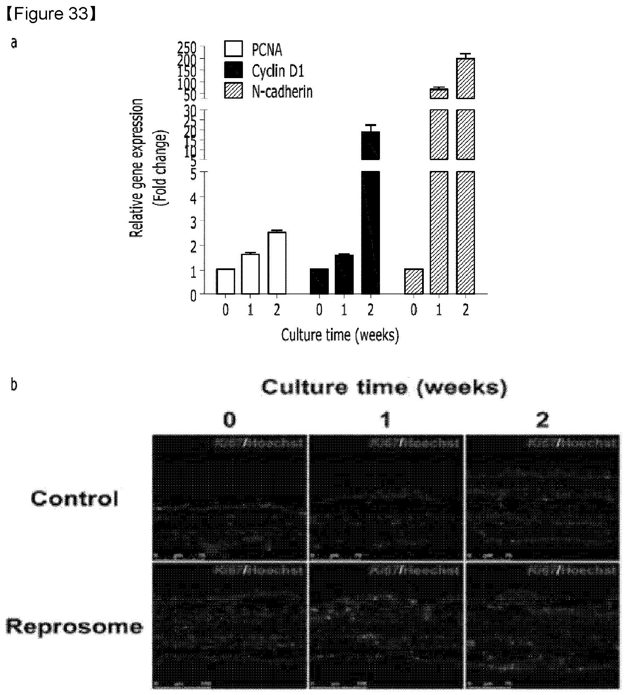

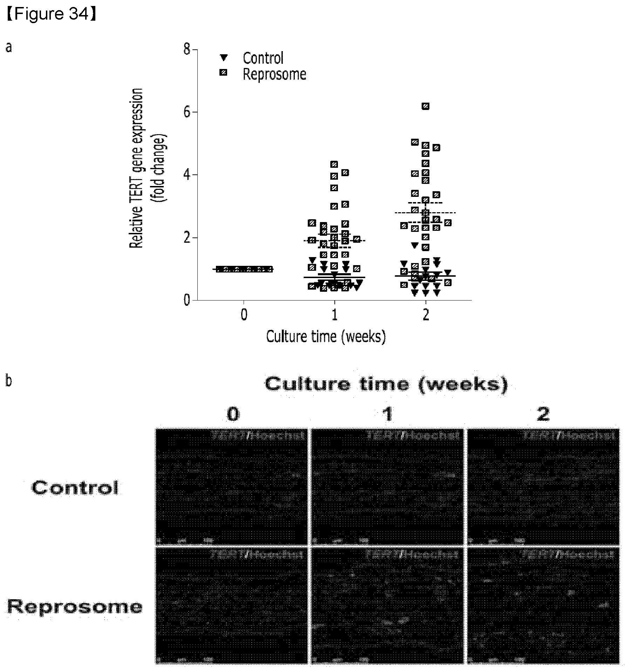

In order to examine how the application of the ultrasound-induced exosomes to animals has on the telomere length, 1×10⁹ and 1×10¹⁰ exosomes produced according to Example 5 were applied to one ear of 10-week-old ICR mice three times a week, and ear samples were collected at 1 week and 2 weeks, respectively, and the telomere length and the TERT RNA expression level therein were analyzed. At this time, as a control, the opposite ear to which D-PBS containing the exosomes diluted therein was applied was used, and the control is indicated as 0 in FIG. 31. As a result of the experiment, it was confirmed that the telomere length in the ear to which the exosomes increased over time, and in particular, as the amount of exosomes applied increased, the increment in the telomere length increased, and expression of TERT in the exosome-treated group relatively increased (FIG. 31). As a result of performing FISH analysis of telomeres and immunofluorescence staining for TERT in the ear tissue treated with 1×10⁹ and 1×10¹⁰ exosomes produced according to Example 5, it could be confirmed that the fluorescence signal intensity and the frequency of TERT increased as the culture time increased (FIGS. 32 and 34). In addition, as a result of analyzing the RNA expression levels of PCNA, cyclin D1 and N-cadherin, which are genes related to cell proliferation, by qPCR, it could be confirmed that the RNA expression levels of the three genes increased. Furthermore, as a result of immunofluorescence staining for Ki67, the fluorescence single intensity and the frequency increased as the culture time increased (FIG. 33).

### Experimental Example 19. Analysis of Skin Regeneration, Wound Healing and Scar Healing Effects of Application of Ultrasound-Induced Exosomes

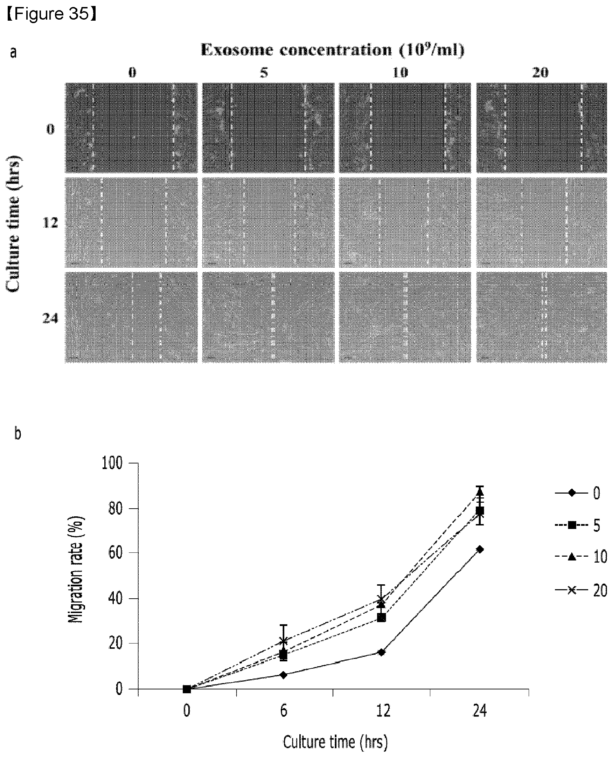

As described above, it was found that the exosomes produced according to the production method of the present invention exhibit effects such as telomere elongation and cell proliferations. Since these effects are highly correlated with cell regeneration, the effects of these exosomes on skin regeneration, wound healing and scar healing were evaluated. First, in order to examine what effect the exosomes have on the migration of cells, the exosomes produced according to Example 5 were added to a medium, and HDFs were cultured in the medium to fill the plate, and then wound healing assay was performed by creating lines by a 20 µl yellow tip to separate the cells at a distance from one another, and then treating the cells with the exosomes at concentrations of 0, 5, 10 and 20 (×10⁹) exosomes/ml, followed by culture. As a result, it was confirmed that the empty space in the exosome-treated group was filled relatively quickly as the exosome concentration increased (FIG. 35), suggesting that the exosomes produced according to the production method of the present invention increased the migration rate of the cells.

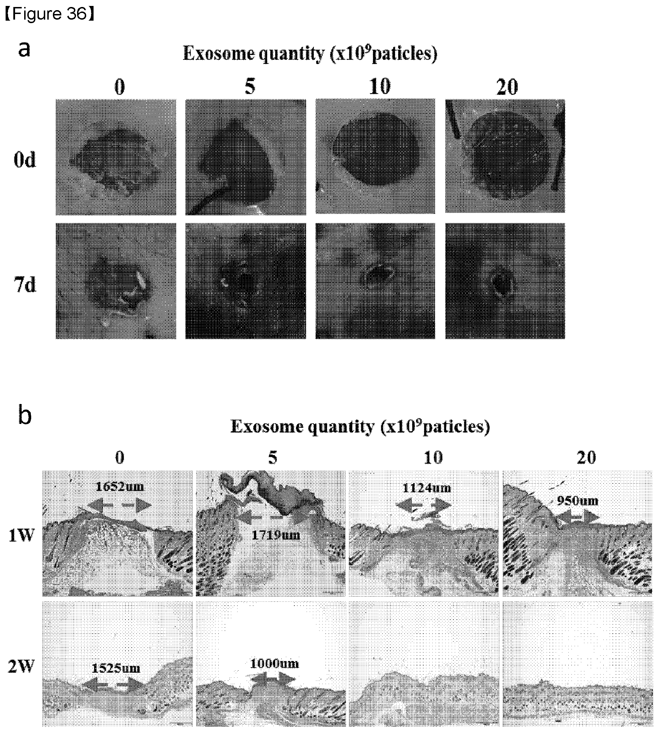

Next, in order to evaluate the effects of the exosome *in vivo,* a 6-mm wound was created on the skin of each 6-week-old C57 mouse using a punch, and the exosomes were diluted in PBS at concentrations of 0, 5, 10 and 20 × 10⁹ exosomes/ml and applied to each wound site twice a week. As a result, it was confirmed that the size of the externally exposed wound (opening) in the exosome-treated mice after 1 week significantly decreased compared to that in the untreated mice. In addition, after 1 week and 2 weeks, the tissue section of each wound site was analyzed by H & E staining. As a result, it could be confirmed that the wound size in the exosome-treated mice was smaller than that in the non-exosome-treated mice, and thus skin regeneration, wound healing and scar healing occurred more quickly in the exosome-treated mice compared to the non-exosome-treated mice. In particular, when the wound site was treated with the exosomes at a concentration of 10 or 20 × 10⁹ exosomes/ml, the skin tissue was completely recovered at week 2, and thus the epidermis, dermal layer, fat layer and muscle layer constituting the skin tissue were regenerated so that they could be clearly distinguished from one another. In addition, in this case, hair follicles, which are skin appendages, were located in the dermal layer and the fat layer without any difference between the wound site and the surrounding area (FIG. 36).

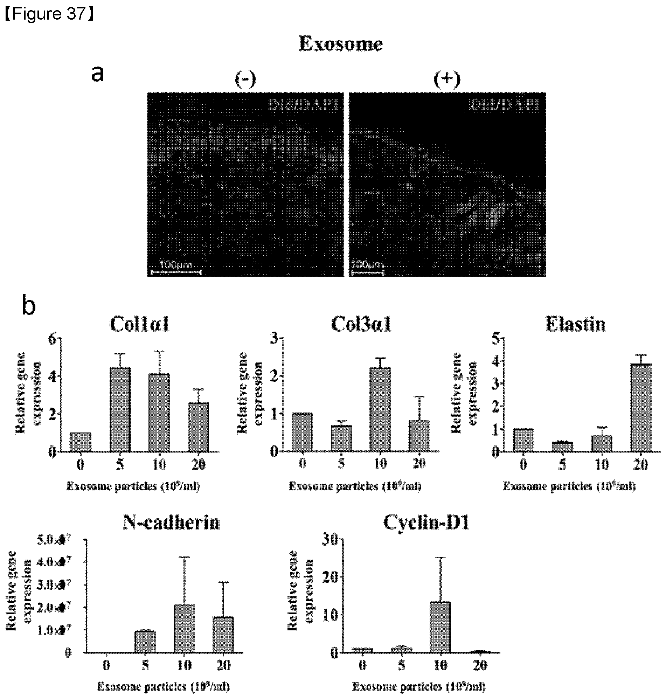

In addition, the exosomes were stained with Vybrant^{™}DiD cell-labeling solution and the skin was treated with the stained exosomes, and after 2 weeks, the site treated with the stained was analyzed under a fluorescence microscope. As a result, it could be confirmed that the exosomes penetrated the entire wound site, and the above-described effects could appear due to this penetration into the entire wound site (FIG. 37). Next, expression of the tissue regeneration- and wound healing-related genes Col1α1, Col3α1, ELN, N-cadherin and cyclin-D1 in the wound sites treated with the exosomes at concentrations of 0, 5, 10 and 20 (×10⁹) exosomes/ml was analyzed by qRT-PCR, and as a result, it was confirmed that expression of one or more of the genes in the experimental group treated with the exosomes was generally higher than that in the control group (FIG. 37). At this time, the gene with the greatest increase in expression was different between the exosome concentrations, and it was difficult to confirm the correlation between the concentration and the increase or decrease in expression.

Taken together, it could be confirmed that the exosomes produced by the production method of the present invention could have skin regeneration, wound healing and scar healing effects not only *in vitro* but also *in vivo.*

### Experimental Example 20. Analysis of Effect by Application of Ultrasound-Induced Exosomes to Cells Derived from Progeria Patient

As described above, it was found that the exosomes produced according to the method of the present invention induced expression of various genes related to anti-aging. To examine these exosomes can have an effect even on senescence-accelerated cells, fibroblasts derived from a patient with progeria were treated with the exosomes, and the telomere length, telomerase activity, TERT expression, cell proliferation, Ki67 expression, and senescence-related β-galactosidase activity in the cells were analyzed. The cells used herein were cells isolated from the thigh skin of a patient with Hutchinson-Gilford progeria syndrome (White/8 years old/male) (AG06297, Coriell Institute, Camden, USA). The cells were treated with the exosomes at a concentration of 1×10⁹ exosomes/ml, and the cells were analyzed 14 days after the initial treatment. The experiment was conducted on the following two groups: an experimental group (ReprosomeX1) treated once at the beginning; and an experimental group (ReprosomeX∞, treated 4 times for 14 days) using a medium containing the above-described concentration of exosomes at every medium replacement.

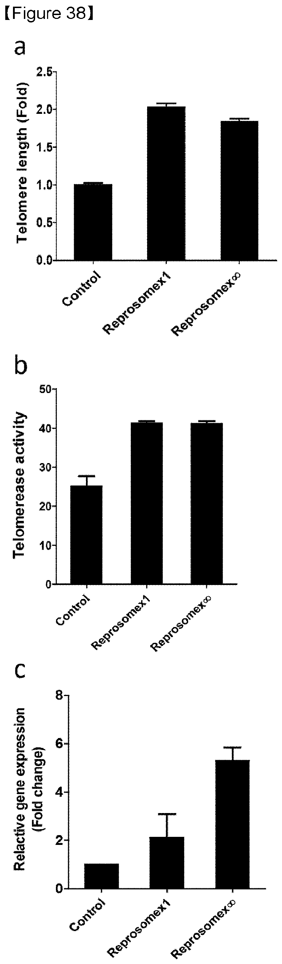

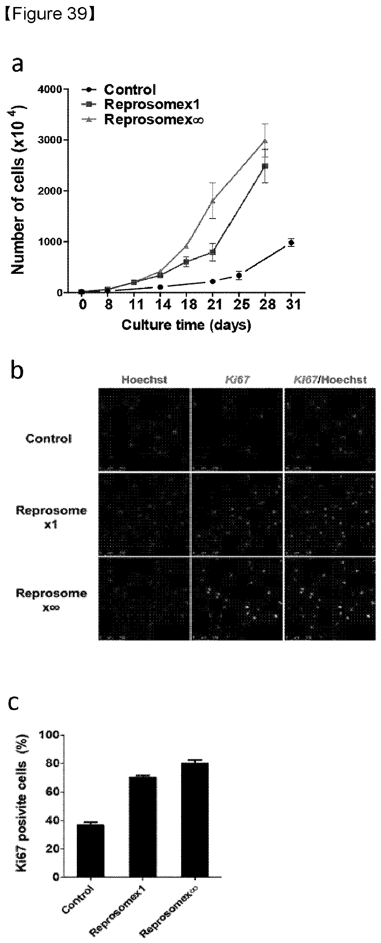

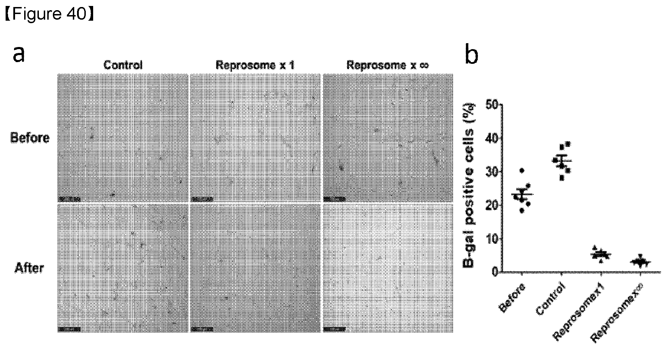

As a result, it could be confirmed that, in both the experimental group treated with the exosomes once at the beginning and the experimental group continuously treated with the exosomes, telomeres were elongated, telomerase activity increased, TERT gene expression increased (FIG. 38), cell proliferation increased and Ki67 protein expression increased (FIG. 39), and β-galactosidase activity decreased (FIG. 40). This suggests that, even when progeria patient cells are treated only once with the exosomes, the exosomes have an anti-senescence effect against the accelerated senescence of the cells.

The above description of the present invention is exemplary, and those of ordinary skill in the art will appreciate that the present invention can be easily modified into other specific forms in accordance with the appended claims. For example, each component described to be of a single type can be implemented in a distributed manner. Likewise, components described to be distributed can be implemented in a combined manner.

The scope of the present invention is defined by the appended claims, and it shall be understood that all modifications and alterations conceived from the meaning and scope of the claims and equivalents thereto are included in the scope of the present invention.

### [Industrial Applicability]

The exosomes for elongating telomeres of cells obtained by the method of the present invention elongate telomeres, induce cell division, have anti-aging and tissue regeneration, and thus may be used as drugs that may prevent, ameliorate and treat not only problems caused by shortening of telomeres, but also various diseases and conditions associated with aging and tissue regeneration. The exosomes and the composition containing the same may exhibit their effects even when they are applied in small amounts in a noninvasive manner.

The method for producing exosomes for elongating telomeres of cells according to the present invention is a method capable of loading various factors having the above-described effects into exosomes, which can penetrate cells, by simply treating cells with physical stimulation. Through this method, exosomes containing high concentrations of active ingredients may be obtained in high yield.

## Claims

1. A method for producing exosomes for elongating telomeres of cells, the method comprising steps of:
a) providing direct ultrasound stimulation to cells at 0.1 to 3 W/cm² and a frequency of 20 kHz to 20 MHz for a duration of 0.1 seconds to 20 minutes;
b) culturing a mixture of the cells provided in step a) and a medium for a predetermined time; and
c) isolating exosomes from the mixture,
where in step a) the providing direct ultrasound stimulation to cells is applying the ultrasound stimulation to a medium containing the cells;
wherein an expression level of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the isolated exosomes is higher than that in the exosomes secreted from the cells before being subjected to the method.

2. The method of claim 1, wherein the exosomes contain 150 ng or more of total RNA in 1 × 10⁸ exosome particles.

3. The method of claim 1, wherein the exosomes contain 1 µg or more of total protein in 1 x 10⁸ exosome particles.

4. The method of claim 1, wherein, when cells are treated with the exosomes, telomerase activity in the cells increases.

5. The method of claim 1, wherein, when cells are treated with the exosomes, β-galactosidase activity in the cells decreases.

## Patentansprüche

1. Verfahren zum Erzeugen von Exosomen zum Verlängern von Telomeren von Zellen, wobei das Verfahren die Schritte umfasst:
a) eines Bereitstellens einer direkten Ultraschallstimulation für Zellen mit 0,1 bis 3 W/cm² und einer Frequenz von 20 kHz bis 20 MHz für eine Dauer von 0,1 Sekunden bis 20 Minuten;
b) eines Kultivierens einer Mischung der in Schritt a) bereitgestellten Zellen und eines Mediums für eine vorgegebene Zeit; und
c) eines Isolierens von Exosomen aus der Mischung,
wobei in Schritt a) das Bereitstellen einer direkten Ultraschallstimulation für Zellen ein Anwenden der Ultraschallsimulation auf ein die Zellen enthaltendes Medium ist,
wobei das Expressionsniveau von einem oder mehreren von TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 und β-Catenin-Genen in den isolierten Exosomen höher ist als jenes in den Exosomen, die aus den Zellen abgesondert wurden, bevor sie dem Verfahren unterzogen werden.

2. Verfahren nach Anspruch 1, wobei die Exosomen 150 ng oder mehr an Gesamt-RNA in 1 × 10⁸ Exosomenpartikeln enthalten.

3. Verfahren nach Anspruch 1, wobei die Exosomen 1 µg oder mehr an Gesamtprotein in 1 × 10⁸ Exosomenpartikeln enthalten.

4. Verfahren nach Anspruch 1, wobei, wenn Zellen mit den Exosomen behandelt werden, die Telomeraseaktivität in den Zellen zunimmt.

5. Verfahren nach Anspruch 1, wobei, wenn Zellen mit den Exosomen behandelt werden, die β-Galactosidaseaktivität in den Zellen abnimmt.

## Revendications

1. Procédé de production d'exosomes pour allonger les télomères de cellules, le procédé comprenant les étapes consistant à :
a) fournir une stimulation ultrasonore directe aux cellules à 0,1 à 3 W/cm² et à une fréquence de 20 kHz à 20 MHz pendant une durée de 0,1 seconde à 20 minutes ;
b) mettre en culture un mélange des cellules fournies dans l'étape a) et un milieu pendant une durée prédéterminée ; et
c) isoler des exosomes à partir du mélange,
où, dans l'étape a), la fourniture d'une stimulation ultrasonore directe aux cellules consiste à appliquer la stimulation ultrasonore à un milieu contenant les cellules ;
dans lequel un niveau d'expression d'un ou de plusieurs parmi les gènes TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 et de la β-caténine dans les exosomes isolés est supérieur à celui dans les exosomes sécrétés par les cellules avant d'être soumises au procédé.

2. Procédé selon la revendication 1, dans lequel les exosomes contiennent 150 ng ou plus d'ARN total dans 1 × 10⁸ particules d'exosome.

3. Procédé selon la revendication 1, dans lequel les exosomes contiennent 1 µg ou plus de protéines totales dans 1 × 10⁸ particules d'exosome.

4. Procédé selon la revendication 1, dans lequel, lorsque des cellules sont traitées avec les exosomes, l'activité télomérase dans les cellules augmente.

5. Procédé selon la revendication 1, dans lequel, lorsque des cellules sont traitées avec les exosomes, l'activité β-galactosidase dans les cellules diminue.
